(19) Europäisches Patentamt / European Patent Office / Office européen des brevets

(11) **EP 3 953 683 B1**

(12) **FASCICULE DE BREVET EUROPEEN**

(45) Date de publication et mention de la délivrance du brevet:
**01.03.2023 Bulletin 2023/09**

(21) Numéro de dépôt: **20719588.4**

(22) Date de dépôt: **06.04.2020**

(51) Classification Internationale des Brevets (IPC):
**G01N 21/3504** *(2014.01)* **G01M 3/38** *(2006.01)*

(52) Classification Coopérative des Brevets (CPC):
**G01M 3/22; G01M 3/38; G01N 21/314; G01N 21/3504;** G01N 33/0027; G01N 2021/1793; G01N 2021/3531

(86) Numéro de dépôt international:
**PCT/EP2020/059746**

(87) Numéro de publication internationale:
**WO 2020/207962 (15.10.2020 Gazette 2020/42)**

(54) **DETECTEUR DE GAZ**

GASSENSOR

GAS SENSOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2019 FR 1903745**

(43) Date de publication de la demande:
**16.02.2022 Bulletin 2022/07**

(73) Titulaires:
• **Office National d'Etudes et de Recherches Aérospatiales**
  **91120 Palaiseau (FR)**
• **TotalEnergies OneTech**
  **92400 Courbevoie (FR)**

(72) Inventeurs:
• **FOUCHER, Pierre-Yves**
  **31320 PECHBUSQUE (FR)**
• **DRUART, Guillaume**
  **91120 PALAISEAU (FR)**

(74) Mandataire: **Plasseraud IP**
  **66, rue de la Chaussée d'Antin**
  **75440 Paris Cedex 09 (FR)**

(56) Documents cités:
**WO-A1-2015/166265 US-A1- 2016 097 713 US-A1- 2018 011 009**

**Description**

**Domaine technique**

L'invention concerne un détecteur de gaz, par exemple pour détecter des fuites d'un gaz tel que le méthane dans un environnement quelconque, notamment en extérieur.

**Technique antérieure**

De nombreuses situations requièrent de détecter si un gaz identifié est présent dans une zone d'espace, que ce soit dans un environnement extérieur ou à l'intérieur d'un volume fermé, dans un bâtiment ou dans une mine par exemple. Ce peut être pour confirmer la sécurité d'un site qui est susceptible de contenir des quantités d'un gaz dangereux ou toxique, ou pour rechercher l'existence d'une fuite accidentelle dans une canalisation d'acheminement ou de distribution d'un gaz. En particulier, de tels besoins de détection concernent des installations d'acheminement et de distribution de méthane ($CH_4$), mais ils existent aussi pour d'autres gaz en fonction des applications concernées.

Une façon connue de détecter le gaz consiste à utiliser un instrument d'imagerie qui est sensible dans une bande spectrale de rayonnement où le gaz est absorbant. Les documents US 2016/097713 et US 2018/011009 en fournissent des descriptions. En général, il s'agit d'une bande infrarouge, pour laquelle l'atmosphère est au moins partiellement transparente, et des images d'une même scène sont saisies répétitivement. Lorsqu'un échappement du gaz se produit dans la scène de sorte qu'une répartition du gaz varie à l'intérieur du champ optique d'entrée de l'instrument d'imagerie, les images qui sont saisies successivement contiennent des zones où la scène est au moins partiellement occultée par le gaz, et les limites de ces zones varient entre images successives. Ce sont alors ces variations qui permettent de confirmer la présence du gaz, à condition que la scène qui est imagée en arrière-plan soit statique.

Toutefois, une telle méthode de détection produit en général un taux de fausses alarmes qui est élevé. Ces fausses alarmes peuvent notamment être dues à l'arrière-plan des images saisies, dont des contributions à ces images sont attribuées par erreur au gaz recherché. Par exemple, de telles contributions peuvent résulter d'un éclairage solaire direct et variable sur des éléments de scène qui sont présents en arrière-plan.

**Problème technique**

A partir de cette situation, un but de la présente invention est de fournir une nouvelle méthode de détection d'un gaz, qui présente un taux réduit de fausses alarmes. Notamment, une telle méthode est désirée, qui permette de discerner plus efficacement le gaz recherché par rapport à des éléments de scène présents en arrière-plan, tels que pouvant exister dans des environnements variés, extérieurs ou intérieurs.

Un autre but de l'invention est de disposer d'une méthode quantitative de détection du gaz, qui permette d'évaluer une quantité du gaz présente dans une zone d'espace, plutôt qu'une méthode limitée à l'évaluation de variations temporelles de cette quantité.

Un but complémentaire est de proposer une telle méthode de détection du gaz qui puisse être mise en oeuvre facilement sur site, c'est-à-dire à l'endroit où la présence du gaz est suspectée. En particulier, l'invention a pour but de proposer un détecteur de gaz qui soit peu lourd et peu encombrant, notamment pour être facilement portatif ou embarqué à bord d'un drone.

Enfin, encore un but de l'invention est de proposer une méthode de détection du gaz qui fournisse un résultat d'évaluation quantitative en temps réel ou quasiment en temps réel.

**Résumé de l'invention**

Pour atteindre l'un au moins de ces buts ou un autre, l'invention propose un nouveau détecteur de gaz pour révéler au moins un gaz qui est appelé gaz-cible et qui est susceptible d'être présent dans un champ optique d'entrée. Ce détecteur de gaz comprend :

- une unité de saisie d'images, qui comprend au moins deux canaux optiques disposés en parallèle pour saisir séparément et simultanément des images respectives d'un même contenu du champ optique d'entrée, appelées images spectrales, dans des bandes spectrales différentes auxquelles lesdits canaux sont dédiés un-à-une ;
- des premiers moyens d'acquisition, adaptés pour fournir une valeur de température ambiante, destinée à être attribuée à une quantité du gaz-cible qui est présente dans le champ optique d'entrée entre une scène d'arrière-plan et l'unité de saisie d'images ;
- des seconds moyens d'acquisition, adaptés pour fournir, pour au moins une des bandes spectrales appelée bande d'analyse, des valeurs de température de brillance d'arrière-plan qui sont destinées à être attribuées à des éléments de la scène d'arrière-plan ;
- une unité de traitement d'images, agencée pour recevoir les images spectrales saisies par chaque canal de l'unité de saisie d'images, et adaptée pour déduire, séparément pour chaque bande d'analyse, une évaluation d'un coefficient de transmission de rayonnement qui est relatif à cette bande d'analyse, et qui est attribué au moins partiellement à une quantité du gaz-cible présente dans une partie du champ optique d'entrée entre les éléments de scène d'arrière-plan et l'unité de saisie d'images, à partir d'une équation qui combine :

  • la valeur de température ambiante,
  • la valeur de température de brillance d'arrière-plan attribuée aux éléments de scène d'arrière-plan dans la partie concernée du champ optique d'entrée, pour la bande d'analyse concernée, et
  • au moins une valeur de température de brillance, appelée valeur de température de brillance apparente, qui correspond à l'image spectrale saisie pour la bande d'analyse, dans la partie concernée du champ optique d'entrée ; et

- une unité de calcul, adaptée pour déduire une évaluation de la quantité de gaz-cible qui est présente dans la partie du champ optique d'entrée, à partir de la valeur du coefficient de transmission de rayonnement qui est relatif à la bande d'analyse.

En particulier, la partie du champ optique d'entrée pour laquelle est évaluée la quantité de gaz-cible qui y est présente, peut correspondre à un point d'image (pixel) de l'image spectrale qui a été saisie pour cette bande d'analyse. Alternativement, elle peut correspondre à une réunion de plusieurs points d'image, ceux-ci pouvant être adjacents les uns aux autres.
Selon l'invention, les premiers moyens d'acquisition sont adaptés pour fournir la valeur de température ambiante selon l'une des façons suivantes :

- à partir d'au moins une partie d'une image spectrale qui a été saisie par un des canaux de l'unité de saisie d'images, cette partie d'image spectrale correspondant à un secteur du champ optique d'entrée déclaré ou considéré comme étant dépourvu d'éléments de scène d'arrière-plan ; et
- à partir d'au moins une partie d'une image spectrale qui a été saisie par un des canaux de l'unité de saisie d'images, dont la bande spectrale associée est contenue dans un domaine spectral d'opacité complète d'un gaz qui est présent dans un secteur du champ optique d'entrée correspondant à la partie d'image spectrale.

Ainsi, grâce à l'invention, il n'est pas nécessaire que le détecteur de gaz incorpore un capteur de température additionnel, notamment un tel capteur de température qui réalise une mesure thermique. La conception du détecteur de gaz est ainsi simplifiée. En outre, étant donné que la valeur de température ambiante est obtenue d'une façon similaire à chaque valeur de température de brillance de bande d'analyse, une meilleure concordance de valeurs en résulte, qui améliore la fiabilité et la précision de la détection du gaz-cible.
Selon une caractéristique supplémentaire de l'invention, le détecteur de gaz est adapté pour saisir l'image spectrale dont une partie au moins est utilisée pour fournir la valeur de température ambiante, en même temps ou lors d'une même séquence d'acquisition d'image, que l'image spectrale de chaque bande d'analyse, lorsque l'unité de traitement d'images combine ensuite cette valeur de température ambiante avec au moins une valeur de température de brillance de l'image spectrale de la bande d'analyse. Ainsi, chaque séquence de fonctionnement du détecteur de gaz peut être suffisante et autonome pour fournir une évaluation de la quantité du gaz-cible qui est présente dans la partie du champ optique d'entrée, sans nécessité d'avoir recours à une ou plusieurs image(s) spectrale(s) saisies(s) antérieurement. Des capacités de mémoire du détecteur de gaz peuvent ainsi être réduites, et une cohérence temporelle est assurée entre des contenus respectifs d'images spectrales qui sont combinées pour obtenir une évaluation de quantité du gaz-cible.
Dans différents modes de réalisation de l'invention, l'unité de saisie d'images peut comprendre par exemple deux, quatre, six, neuf, douze ou seize canaux optiques qui sont disposés en parallèle, sans limitation mais de préférence au plus vingt canaux.
Une équation qui peut être utilisée par l'unité de traitement d'images pour évaluer le coefficient de transmission du rayonnement qui est relatif à la bande d'analyse, et qui est attribué au moins partiellement au gaz-cible, peut être de la forme :

$$\tau_{bande\_1} = 1 + (TB_{apparente\_1} - TB_{arrière\text{-}plan\_1})/(TB_{arrière\text{-}plan\_1} - T_{ambiante}),$$

où

$\tau_{bande\_1}$ est la valeur du coefficient de transmission du rayonnement qui est relatif à la bande d'analyse, notée bande_1, et qui est attribué au moins partiellement au gaz-cible présent dans la partie du champ optique d'entrée,

$T_{ambiante}$ est la valeur de température ambiante qui est fournie par les premiers moyens d'acquisition,

$TB_{arrière-plan\_1}$ est la valeur de température de brillance d'arrière-plan qui est fournie par les seconds moyens d'acquisition, et qui est attribuée aux éléments de scène d'arrière-plan contenus dans la partie du champ optique d'entrée, pour la bande d'analyse bande_1, et

$TB_{apparente\_1}$ est la valeur de température de brillance apparente qui correspond à l'image spectrale saisie pour la bande d'analyse bande_1, dans la partie du champ optique d'entrée.

Une telle équation est simple et rapide à mettre en oeuvre, et compatible avec l'obtention d'un résultat quantitatif de détection du gaz-cible en temps réel ou quasiment en temps réel. Lorsque la valeur qui est ainsi obtenue pour le coefficient de transmission $\tau_{bande\_1}$ n'est pas contenue entre 0 et 1, ces deux limites étant admises, le résultat peut être écarté. Une telle incohérence peut avoir été provoquée par une évolution de l'arrière-plan entre un instant pour lequel la température de brillance d'arrière-plan a été déterminée et l'instant de saisie de l'image spectrale utilisée pour déterminer la température de brillance apparente. Elle peut aussi avoir été provoquée par une évolution de la température ambiante entre un instant pour lequel cette température ambiante a été déterminée et l'instant de saisie de l'image spectrale utilisée pour déterminer la température de brillance apparente. Une autre raison encore peut être la présence d'un autre gaz dans la partie du champ optique d'entrée, qui a contribué à la luminance saisie dans l'image spectrale, ou qui est intervenu dans le fonctionnement des premiers et/ou seconds moyens d'acquisition. Ces causes d'incohérence pourront être réduites en utilisant des premiers et seconds moyens d'acquisition qui produisent des données relatives au même instant que celui de saisie de chaque image spectrale de bande d'analyse.

Avantageusement, lorsque le détecteur de gaz comporte plusieurs bandes d'analyse, l'équation précédente pour évaluer le coefficient de transmission du rayonnement peut être utilisée séparément pour chacune de ces bandes d'analyse, pour évaluer le coefficient de transmission du rayonnement qui est relatif à cette bande d'analyse indépendamment de chaque autre bande d'analyse. L'utilisation de plusieurs bandes d'analyse permet, de façon générale pour l'invention, d'obtenir une évaluation qui est plus fiable pour la quantité du gaz-cible.

Préférablement, l'unité de traitement d'images peut être adaptée en outre pour appliquer à chaque image spectrale, une correction de valeurs de luminance telles que saisies par l'unité de saisie d'images, afin de prendre en compte au moins un composant atmosphérique qui est présent dans le champ optique d'entrée. Les valeurs de luminance corrigées sont alors utilisées par l'unité de traitement d'images pour évaluer le coefficient de transmission du rayonnement relatif à chaque bande d'analyse. Le composant atmosphérique qui peut ainsi être pris en compte peut être la vapeur d'eau, et/ou éventuellement le dioxyde de carbone.

L'unité de calcul peut être adaptée pour déterminer la quantité du gaz-cible qui est présente dans la partie du champ optique d'entrée, notée $Q_{gaz-cible}$, en inversant une équation qui relie la valeur du coefficient de transmission de rayonnement à cette quantité du gaz-cible, pour chaque bande d'analyse. En effet, dans un cas simplifié de répartition uniforme du gaz-cible à l'intérieur d'un panache, l'équation peut être du type : $\tau_{bande\_1} = \exp(-Q_{gaz-cible} \cdot Abs_{bande\_1})$, où $Abs_{bande\_1}$ est l'absorbance du gaz-cible dans la bande d'analyse bande_1. Toutefois, un calcul plus précis peut être effectué pour tenir compte de variations plus complexes de la concentration du gaz-cible dans le panache. Il est alors préférable de disposer pour chaque bande d'analyse, d'une table de valeurs du coefficient de transmission de rayonnement en fonction de profils de la concentration du gaz-cible le long du trajet de rayonnement qui relie un élément de scène d'arrière-plan à l'unité de saisie d'images. Pour cela, l'unité de calcul peut avantageusement être agencée pour déterminer la quantité du gaz-cible qui est présente dans la partie du champ optique d'entrée en comparant les valeurs des coefficients de transmission de rayonnement qui ont été déduites par l'unité de traitement d'images séparément pour plusieurs bandes d'analyse, à des valeurs de ces coefficients de transmission de rayonnement qui ont été prédéterminées pour les mêmes bandes d'analyse. Des erreurs susceptibles d'affecter les résultats numériques obtenus pour la concentration du gaz-cible peuvent être réduites de cette façon. Les valeurs prédéterminées, possiblement par des calculs préalables, pour les coefficients de transmission de rayonnement peuvent alors être enregistrées dans une unité de stockage de données qui est accessible par l'unité de calcul. Elles sont relatives à des profils variables de la concentration du gaz-cible sur le trajet de rayonnement qui relie un élément de scène d'arrière-plan à l'unité de saisie d'images. La valeur de quantité du gaz-cible correspond alors à une intégration spatiale du profil de concentration du gaz-cible sur le trajet du rayonnement entre l'élément de scène d'arrière-plan et l'unité de saisie d'images. Une telle utilisation de valeurs prédéterminées, sous forme de table indexée en fonction des profils de concentration du gaz-cible, permet de réduire les calculs à effectuer lors de chaque séquence de détection du gaz-cible. De cette façon, le résultat quantitatif de détection du gaz-cible peut être obtenu en temps réel ou quasiment en temps réel, pour chaque séquence de détection qui est effectuée.

De préférence, les bandes spectrales des canaux de l'unité de saisie d'images peuvent être telles que le gaz-cible possède des valeurs d'absorbance qui sont différentes entre deux bandes spectrales différentes. Alternativement ou en combinaison, les bandes spectrales des canaux de l'unité de saisie d'images peuvent être telles que le gaz-cible possède des valeurs d'un quotient qui sont différentes entre deux bandes spectrales différentes, le quotient étant calculé pour chaque bande spectrale comme la valeur d'absorbance du gaz-cible dans cette bande spectrale, divisée par la valeur d'absorbance d'au moins un composant atmosphérique présent dans le champ optique d'entrée, dans la même bande spectrale.

Lorsque la valeur de température ambiante est fournie par les premiers moyens d'acquisition à partir d'une partie d'image spectrale qui correspond à un secteur du champ optique d'entrée déclaré ou considéré comme étant dépourvu d'éléments de scène d'arrière-plan, l'unité de saisie d'images peut être orientée pour saisir cette image spectrale de sorte que le secteur du champ optique d'entrée soit au moins partiellement occupé par une zone de ciel, sans élément de scène intermédiaire entre la zone de ciel et l'unité de saisie d'images.

De façon générale, la valeur de température ambiante peut correspondre à une valeur de température de brillance d'un composant d'atmosphère qui est déduite de la partie d'image spectrale concernée pour la bande spectrale du canal utilisé.

Les seconds moyens d'acquisition peuvent être adaptés pour fournir les valeurs de température de brillance d'arrière-plan selon l'une des façons suivantes :

- à partir d'au moins une image spectrale qui contient un des éléments de scène d'arrière-plan, qui a été saisie par le canal de l'unité de saisie d'images dédié à la bande d'analyse, et qui est déclarée ou considérée comme ayant été saisie alors que le champ optique d'entrée ne contenait pas de gaz-cible ;
- à partir d'au moins une image spectrale qui contient un des éléments de scène d'arrière-plan, qui a été saisie par un des canaux de l'unité de saisie d'images pour lequel la bande spectrale correspondante, dite bande de référence, est contenue dans un domaine spectral de transparence du gaz-cible, ou dans laquelle bande de transparence le gaz-cible possède une transparence plus grande que dans chaque bande d'analyse, l'unité de traitement d'images étant adaptée pour identifier un matériau de l'élément de scène d'arrière-plan à partir de l'image spectrale saisie pour la bande de référence, et pour déduire la valeur de température de brillance d'arrière-plan pour chaque bande d'analyse et pour l'élément de scène d'arrière-plan, à partir de la valeur de température ambiante et d'une valeur d'émissivité spectrale du matériau identifié pour ledit élément de scène d'arrière-plan ; et
- à partir d'au moins une image spectrale qui contient un des éléments de scène d'arrière-plan, qui a été saisie par un des canaux de l'unité de saisie d'images pour lequel la bande spectrale correspondante, dite bande de référence, est contenue dans un domaine spectral de transparence du gaz-cible, ou dans laquelle bande de transparence le gaz-cible possède une transparence plus grande que dans chaque bande d'analyse, l'unité de traitement d'images étant adaptée pour déduire les valeurs de température de brillance d'arrière-plan pour chaque bande d'analyse en utilisant une régression linéaire à partir de valeurs de température de brillance d'arrière-plan déduites de l'image spectrale saisie pour la bande de référence. Selon un perfectionnement, plusieurs régressions linéaires peuvent être mises en oeuvre, séparément à l'intérieur de zones de découpage communes aux images spectrales. Ces zones de découpage peuvent être définies à partir de l'image spectrale qui a été saisie pour la bande de référence, et sont telles que les valeurs de température de brillance dans cette image spectrale varient dans une mesure limitée à l'intérieur de chaque zone de découpage.

Avantageusement, lorsque les seconds moyens d'acquisition sont adaptés en outre pour fournir les valeurs de température de brillance d'arrière-plan à partir d'au moins une image spectrale saisie par un canal de l'unité de saisie d'images qui correspond à une bande de référence, le détecteur de gaz peut être adapté pour saisir l'image spectrale qui correspond à cette bande de référence en même temps ou lors d'une même séquence d'acquisition d'image, que l'image spectrale de chaque bande d'analyse. Ainsi, les avantages suivants de l'invention sont accrus d'une façon supplémentaire :

- chaque séquence de fonctionnement du détecteur de gaz est suffisante et autonome pour fournir une évaluation de la quantité du gaz-cible, sans recours à une ou plusieurs image(s) spectrale(s) saisies(s) antérieurement ;
- les capacités de mémoire du détecteur de gaz peuvent être réduites ; et
- une cohérence temporelle est assurée entre des contenus respectifs d'images spectrales qui sont combinées pour obtenir une évaluation de quantité du gaz-cible.

Dans des modes de réalisation préférés de l'invention, l'unité de saisie d'images peut comprendre un capteur d'images matriciel qui est commun à tous les canaux, et qui est sensible simultanément dans toutes les bandes spectrales de ces canaux. Alors, une partie d'une surface photosensible de ce capteur d'images peut être dédiée à chaque canal, séparément de chaque autre canal. Chaque canal comprend alors, à l'intérieur de l'unité de saisie d'images :

- une partie d'optique qui est agencée pour former une image d'un contenu du champ optique d'entrée sur la partie de surface photosensible dédiée à ce canal, le champ optique d'entrée étant commun à tous les canaux ; et
- des moyens de filtrage spectral, qui sont adaptés pour déterminer la bande spectrale du canal.

Une telle configuration optique permet de réaliser l'unité de saisie d'images sous forme d'un module unique, particulièrement compact et de poids réduit. Le détecteur de gaz peut alors être portatif, et/ou être facilement installé à bord d'un drone. La possibilité d'utilisation à bord d'un drone est particulièrement avantageuse lorsque le gaz à détecter présente un danger, notamment lorsqu'il est toxique.

Le capteur d'images peut être du type capteur quantique et, optionnellement, le détecteur de gaz peut comprendre en outre des moyens de refroidissement qui sont agencés pour refroidir le capteur d'images à une température inférieure à 150 K (kelvin).

De façon générale pour l'invention, chaque canal de l'unité de saisie d'images peut comprendre des moyens de filtrage spectral tels que la bande spectrale de ce canal possède une largeur qui est comprise entre 10 nm (nanomètre) et 500 nm (ou 0,50 $\mu$m), en termes de longueur d'onde du rayonnement. Ces moyens de filtrage spectral peuvent alors être adaptés pour que les bandes spectrales des canaux soient contenues dans un premier domaine spectral qui correspond à des longueurs d'onde de rayonnement comprises entre 7 $\mu$m et 10 $\mu$m. Ce premier domaine spectral est couramment désigné par LWIR, pour «long-wavelength infrared» en anglais. Alternativement, les moyens de filtrage spectral peuvent être adaptés pour que les bandes spectrales des canaux soient contenues dans un second domaine spectral qui correspond à des longueurs d'onde de rayonnement comprises entre 3 $\mu$m et 5 $\mu$m. Ce second domaine spectral est couramment désigné par MWIR, pour «mid-wavelength infrared» en anglais.

Pour un détecteur de gaz qui est conforme à l'invention, adapté pour le méthane en tant que gaz-cible, et qui est fonctionnel dans le domaine LWIR :

- la bande spectrale d'un premier des canaux peut s'étendre autour de 7,7 $\mu$m, avec une largeur de bande spectrale qui est inférieure à 0,35 $\mu$m ;
- la bande spectrale d'un deuxième des canaux peut s'étendre autour de 8,05 $\mu$m, avec une largeur de bande spectrale qui est inférieure à 0,35 $\mu$m ;
- la bande spectrale d'un troisième des canaux, optionnel, peut s'étendre autour de 7,35 $\mu$m, avec une largeur de bande spectrale qui est inférieure à 0,35 $\mu$m ; et
- la bande spectrale d'un quatrième des canaux, aussi optionnel, peut s'étendre autour de 8,35 $\mu$m ou 9,05 $\mu$m, avec une largeur de bande spectrale qui est inférieure à 0,35 $\mu$m.

Pour un autre détecteur de gaz qui est conforme à l'invention, aussi adapté pour le méthane en tant que gaz-cible, mais qui est fonctionnel dans le domaine MWIR :

- la bande spectrale d'un premier des canaux peut s'étendre autour de 3,375 $\mu$m, avec une largeur de bande spectrale qui est inférieure à 0,30 $\mu$m ;
- la bande spectrale d'un deuxième des canaux peut s'étendre autour de 3,225 $\mu$m, avec une largeur de bande spectrale qui est inférieure à 0,30 $\mu$m ;
- la bande spectrale d'un troisième des canaux, optionnel, peut s'étendre autour de 3,05 $\mu$m, avec une largeur de bande spectrale qui est inférieure à 0,30 $\mu$m, et
- la bande spectrale d'un quatrième des canaux, aussi optionnel, peut s'étendre autour de 4,237 $\mu$m ou 3,505 $\mu$m, avec une largeur de bande spectrale qui est inférieure à 0,30 $\mu$m.

Dans certains cas, il peut être difficile de disposer d'un filtre passe-bande pour produire la bande spectrale de l'un au moins des canaux optiques de l'unité de saisie d'images. Alors, il est possible de réaliser ce canal optique sous forme d'un canal optique composite, à partir de deux des canaux optiques de l'unité de saisie d'images qui sont appelés dans ce cas canaux optiques élémentaires. La bande spectrale du canal optique composite résulte alors des positions relatives et des largeurs spectrales des bandes spectrales respectives des deux canaux optiques élémentaires. Le filtre spectral de chaque canal optique élémentaire peut être de type passe-bande, notamment avec une position spectrale et/ou une largeur spectrale qui est (sont) différente(s) de celle(s) de la bande spectrale du canal optique composite, ou être de type passe-haut ou passe-bas. Alors, le détecteur de gaz, notamment les unités de saisie et de traitement d'images, peut être adapté pour combiner les images spectrales qui sont saisies séparément par les deux canaux optiques élémentaires pour un même contenu du champ optique d'entrée, afin d'obtenir une image spectrale qui correspond à la bande spectrale du canal optique composite. En particulier, le détecteur de gaz peut être adapté pour calculer une différence entre les images spectrales qui sont saisies par les deux canaux optiques élémentaires pour le même contenu du champ optique d'entrée, afin d'obtenir l'image spectrale qui correspond à la bande spectrale du canal optique composite.

Enfin, de façon générale, un détecteur de gaz conforme à l'invention peut être adapté pour révéler plusieurs gaz-cibles différents qui sont susceptibles d'être présents simultanément dans le même champ optique d'entrée. Dans ce cas, la bande spectrale de l'un au moins des canaux de l'unité de saisie d'images peut être contenue simultanément dans des domaines spectraux d'absorption respectifs d'au moins deux des gaz-cibles. Ainsi, une même image spectrale qui est saisie par ce canal peut être utilisée par l'unité de traitement d'images et l'unité de calcul pour déduire des évaluations de quantités respectives de ces au moins deux gaz-cibles qui sont présentes dans le champ optique d'entrée. Autrement dit, la même bande spectrale de l'unité de saisie d'images peut servir de bande d'analyse pour lesdits au moins deux gaz-cibles.

**Brève description des figures**

Les caractéristiques et avantages de la présente invention apparaîtront plus clairement dans la description détaillée ci-après d'exemples de réalisation non-limitatifs, en référence aux figures annexées parmi lesquelles :

[Fig. 1] est une vue en coupe d'une unité de saisie d'images qui fait partie d'un détecteur de gaz conforme à l'invention ;

[Fig. 2] est un schéma synoptique de différents éléments d'un détecteur de gaz conforme à l'invention ; et

[Fig. 3] montre une utilisation possible d'un détecteur de gaz conforme à l'invention.

**Description détaillée de l'invention**

Pour raison de clarté, les dimensions des éléments qui sont représentés dans ces figures ne correspondent ni à des dimensions réelles, ni à des rapports de dimensions réels. En outre, des références identiques qui sont indiquées dans des figures différentes désignent des éléments identiques ou qui ont des fonctions identiques.

Dans la suite, l'invention est décrite en détail pour un détecteur de gaz à quatre canaux optiques, et pour un tel détecteur de gaz qui est conçu pour révéler une présence de méthane gazeux dans un environnement terrestre. Les bandes spectrales sont alors sélectionnées non seulement en fonction du gaz-cible à détecter, c'est-à-dire le méthane, mais aussi en fonction des bandes d'absorption des composants de l'atmosphère terrestre. Parmi ces composants de l'atmosphère à prendre en compte, la vapeur d'eau ($H_2O$) est particulièrement importante, mais le dioxyde de carbone ($CO_2$) peut aussi intervenir dans le procédé d'analyse qui est mis en oeuvre dans le détecteur. Toutefois, il est entendu que l'invention n'est pas limitée au méthane en tant que gaz-cible, qu'un nombre différent de canaux optiques peut être utilisé, ce nombre de canaux étant supérieur ou égal à deux et préférablement inférieur à neuf, et que le détecteur peut être adapté pour d'autres environnements que des environnements terrestres.

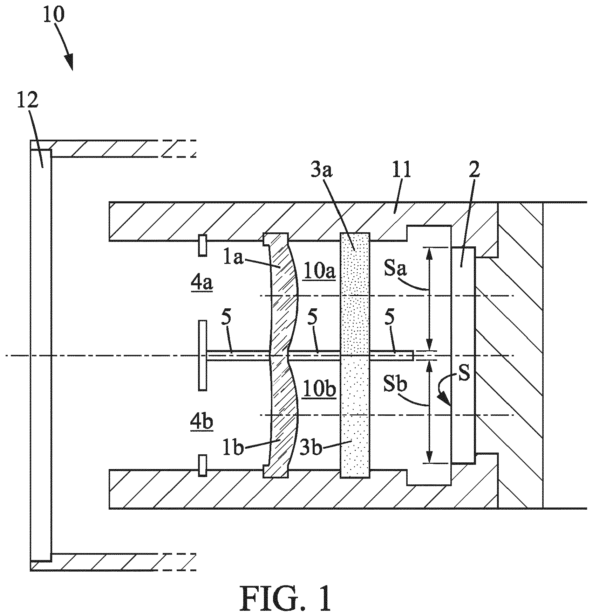

Conformément à [Fig. 1], une unité de saisie d'images 10 peut comprendre une optique de formation d'images et un capteur d'images 2, qui sont assemblés à l'intérieur d'un module unique. Par exemple, une paroi latérale 11 peut maintenir une matrice 2 x 2 de quatre lentilles juxtaposées. Deux de ces lentilles qui sont visibles dans la figure sont référencées 1a, 1b. Toutes les lentilles sont situées à une distance fixe devant la surface photosensible S du capteur d'images 2. Les lentilles 1a, 1b,... définissent chacune un canal optique séparé, en formant des images respectives du contenu d'un même champ optique d'entrée sur des parties disjointes de la surface photosensible S du capteur d'images 2. Les références 10a et 10b désignent les deux canaux optiques qui sont visibles dans la figure, et les références Sa et Sb désignent les parties correspondantes de la surface photosensible S du capteur d'images 2. Chaque voie optique 10a, 10b,... comprend en outre un filtre spectral 3a, 3b,..., respectivement, qui peut être situé entre la lentille du canal optique concerné et le capteur d'images 2. Eventuellement, l'une au moins des surfaces optiques de la lentille 1a, 1b,... de l'un au moins des canaux 10a, 10b,... peut être adaptée en fonction de la bande spectrale de transmission du filtre 3a, 3b,... de ce canal. Pour la configuration de l'unité de saisie d'images 10 qui est représentée, la distance entre chaque lentille 1a, 1b,... et le capteur d'images 2 correspond à la valeur de longueur focale commune à tous les canaux. Ainsi, les quatre canaux optiques 10a, 10b,... forment simultanément quatre images séparées du même contenu du champ optique d'entrée, mais pour des bandes spectrales différentes, telles que déterminées par les filtres 3a, 3b,... Ces images séparées sont appelées images spectrales dans la présente description. Une matrice de diaphragmes d'ouvertures 4a, 4b,... peut en outre être utilisée pour ajuster les uns par rapport aux autres des niveaux d'intensité des images spectrales produites par les quatre canaux 10a, 10b,... Par ailleurs, des séparateurs opaques 5 peuvent être disposés entre les canaux 10a, 10b,... pour supprimer des rayons parasites qui pourraient traverser entre des canaux différents.

A titre d'exemple, les valeurs numériques suivantes peuvent être adoptées pour chaque canal optique de l'unité de saisie d'images 10 :

longueur focale des lentilles : 7 mm (millimètre) environ,
nombre d'ouverture : 3,9 environ,
pas («pitch» en anglais) des photodétecteurs (pixels) dans la surface photosensible S du capteur d'images 2 : 15 μm (micromètre), et
champ de vue : 40° (degré) x 30°.

Ces valeurs correspondent à une résolution angulaire, couramment désignée par IFOV pour «instantaneous field of view» en anglais, de 0,12°, soit 2,1 milliradians. La valeur de longueur focale, en particulier, est compatible avec des dimensions externes de l'unité de saisie d'images 10 qui sont réduites.

Le capteur d'images 2 peut être du type capteur quantique matriciel, par exemple de technologie HgCdTe désignée par MCT pour tellurure de mercure-cadmium, et est sensible dans toutes les bandes spectrales de transmission des filtres 3a, 3b,... Ainsi, à chaque séquence de fonctionnement du capteur d'images 2, il transmet en sortie des données de quatre images spectrales du contenu du champ optique d'entrée, respectivement pour les bandes spectrales des quatre canaux 10a, 10b,... Par exemple, les quatre bandes spectrales peuvent appartenir au domaine LWIR, ou appartenir au domaine MWIR. Dans le cas du domaine LWIR, mais éventuellement aussi pour le domaine MWIR, l'unité de saisie d'images 10 peut être associée à un système de refroidissement, afin de réduire des émissions thermiques de rayonnement par les matériaux des composants de cette unité de saisie d'images, et aussi réduire un bruit photonique du capteur d'images 2 et un bruit de fond instrumental. La température de fonctionnement de l'unité de saisie d'images 10 peut alors être inférieure à 150 K. Lorsqu'un tel système de refroidissement est utilisé, l'unité de saisie d'images 10 est contenue dans une enceinte à vide, appelée couramment cryostat, qui est couplée thermiquement à une machine à froid. La référence 12 désigne un hublot transparent qui peut être disposé devant l'entrée optique de l'unité de saisie d'images 10. Ce hublot 12 fournit une ouverture optique pour l'entrée du rayonnement dans l'unité de saisie d'images 10, tout en assurant une étanchéité du cryostat.

Pour le méthane en tant que gaz-cible, et pour une détection dans le domaine spectral LWIR, un choix possible pour les bandes spectrales des quatre canaux optiques de l'unité de saisie d'images 10 peut être :

pour la bande spectrale bande_1 : centrée à 7,75 $\mu$m avec une largeur de bande spectrale qui peut être égale à 0,30 $\mu$m,
pour la bande spectrale bande_2 : centrée à 8,05 $\mu$m avec une largeur de bande spectrale qui peut être égale à 0,30 $\mu$m,
pour la bande spectrale bande_3 : centrée à 7,35 $\mu$m avec une largeur de bande spectrale qui peut être égale à 0,30 $\mu$m, et
pour la bande spectrale bande_4 : centrée à 8,35 $\mu$m ou 9,05 $\mu$m avec une largeur de bande spectrale qui peut être égale à 0,30 $\mu$m.

Ces valeurs spectrales s'entendent en termes de longueur d'onde, comme toutes les valeurs spectrales qui sont citées dans la présente description. La bande spectrale bande_1 ainsi définie est très sensible à la présence de méthane, correspondant à un domaine d'absorption importante pour ce gaz. Toutefois, elle est aussi très sensible à la présence de vapeur d'eau dans la composition atmosphérique. La bande spectrale bande_2 est aussi sensible au méthane et à la vapeur d'eau, bien que dans des mesures inférieures à celles de la bande spectrale bande_1. La bande spectrale bande_3 est très peu sensible au méthane, et peut être utilisée par les moyens d'acquisition 21 qui sont décrits plus loin. Enfin, la bande spectrale bande_4 est surtout sensible aux émissions thermiques de rayonnement par tous les éléments de scène, dont ceux qui sont présents dans l'arrière-plan. Elle peut donc être utilisée par les moyens d'acquisition 22 qui sont aussi décrits plus loin.

Une autre sélection de bandes spectrales qui est aussi possible pour le méthane en tant que gaz-cible, mais pour une détection dans le domaine spectral MWIR, peut être :

pour la bande spectrale bande_1 : centrée à 3,375 $\mu$m avec une largeur de bande spectrale qui peut être égale à 0,20 $\mu$m,
pour la bande spectrale bande_2 : centrée à 3,225 $\mu$m avec une largeur de bande spectrale qui peut être égale à 0,20 $\mu$m,
pour la bande spectrale bande_3 : centrée à 3,05 $\mu$m avec une largeur de bande spectrale qui peut être égale à 0,20 $\mu$m, et
pour la bande spectrale bande_4 : centrée à 4,237 $\mu$m ou 3,505 $\mu$m avec une largeur de bande spectrale qui peut être égale à 0,20 $\mu$m.

Dans cette autre sélection, la bande spectrale bande_1 est très sensible à la présence de méthane, correspondant encore à un domaine d'absorption importante pour ce gaz. Un avantage de cette bande spectrale est d'être sensible dans une mesure moindre à de la vapeur d'eau qui est présente dans la composition atmosphérique. Toutefois, elle est en général très sensible à une incidence directe d'un flux solaire sur les éléments de scène d'arrière-plan. La bande spectrale bande_2 est aussi sensible au méthane, bien que dans une mesure inférieure à celle de la bande bande_1. Elle est plus sensible à la présence de vapeur d'eau que la bande spectrale bande_1, mais moins sensible à l'incidence directe de flux solaire, encore en comparaison avec la bande spectrale bande_1. La bande spectrale bande_3 est peu sensible à la présence de méthane, mais très sensible à la présence de vapeur d'eau. Elle peut alors être utilisée par les moyens d'acquisition. Enfin, la bande spectrale bande_4 est très peu sensible au méthane à 4,237 $\mu$m et à 3,505 $\mu$m. A 4,237 $\mu$m, elle est surtout sensible au dioxyde de carbone présent dans la composition atmosphérique, et peut aussi être utilisée par les moyens d'acquisition 21. A 3,505 $\mu$m, la bande spectrale bande_4 est surtout sensible aux éléments de scène d'arrière-plan, et peut être utilisée par les moyens d'acquisition 22.

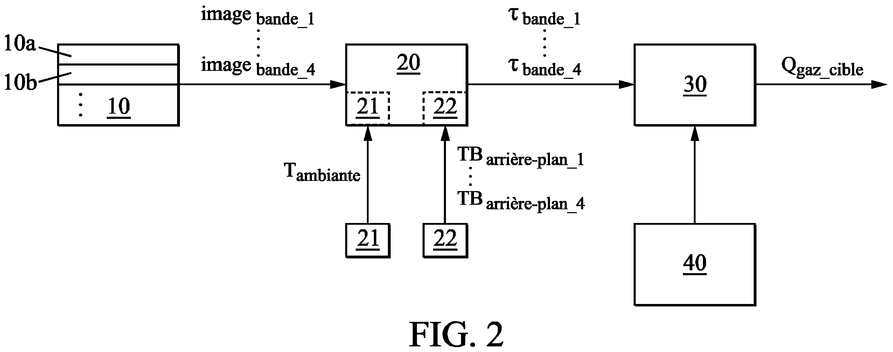

Conformément à [Fig. 2], le détecteur de gaz comprend l'unité de saisie d'images 10, une unité de traitement d'images 20 et une unité de calcul 30, qui sont connectées entre elles pour permettre les transferts de données suivants :

- de l'unité de saisie d'images 10 à l'unité de traitement d'images 20 : les images qui sont saisies simultanément dans les différentes bandes spectrales, notées $image_{bande\_1}$,..., $image_{bande\_4}$ dans le cas de l'unité de saisie d'images à quatre canaux optiques de [Fig. 1], bande_1,..., bande_4 désignant encore les quatre bandes spectrales. Ainsi, $image_{bande\_1}$ a été saisie en filtrant le rayonnement conformément à la bande spectrale bande_1, par exemple par le canal optique 10a,..., $image_{bande\_4}$ a été saisie en filtrant le rayonnement conformément à la bande spectrale bande_4, par l'un des canaux optiques autre que celui de $image_{bande\_1}$ ;
- de l'unité de traitement d'images 20 à l'unité de calcul 30 : des valeurs de coefficients de transmission de rayonnement, notées $\tau_{bande\_1}$,..., $\tau_{bande\_4}$, relatifs séparément aux bandes spectrales bande_1,..., bande_4. Chaque valeur d'un des coefficients de transmission concerne en outre une partie identifiée à l'intérieur du champ optique d'entrée de l'unité de saisie d'images 10. Par exemple, une valeur de chacun des coefficients de transmission $\tau_{banda\_1}$,..., $\tau_{bande\_4}$ est déterminée séparément pour chaque point d'image, ou pixel, de chaque image spectrale $image_{bande\_1}$,..., $image_{bande\_4}$ ; et
- en sortie de l'unité de calcul 30 : une valeur $Q_{gaz\text{-}cible}$ de la quantité du gaz-cible qui est contenue dans chaque partie identifiée du champ optique d'entrée.

La référence 21 désigne des moyens pour obtenir et transmettre à l'unité de traitement d'images 20, une valeur de température ambiante $T_{ambiante}$. Cette valeur $T_{ambiante}$ est relative à l'atmosphère gazeuse qui est présente dans le champ optique d'entrée de l'unité de saisie d'images 10, et aussi relative au gaz-cible qui peut y être présent. En effet, du gaz-cible qui peut provenir d'un point de source avec une température différente parvient très rapidement à un équilibre thermique avec l'atmosphère environnante. Le gaz-cible possède donc essentiellement la valeur de température $T_{ambiante}$ à l'intérieur du champ optique d'entrée, sauf éventuellement dans un voisinage très proche de son point de source, qui est négligeable la plupart des fois par rapport au volume de panache de gaz-cible déjà apparu.

Les moyens 21 qui fournissent la valeur $T_{ambiante}$ de température ambiante ont été appelés premiers moyens d'acquisition dans la partie générale de la présente description. Ils pourraient être constitués, en principe, par un capteur thermique de température qui serait en contact avec l'atmosphère, tel qu'un thermomètre. Dans un tel cas, qui ne correspond pas à la présente invention, les moyens 21 seraient externes à l'unité de traitement d'images 20, et connectés à celle-ci pour transmettre la valeur $T_{ambiante}$.

Dans l'invention, la valeur $T_{ambiante}$ est déduite conformément à la loi de Planck, à partir de l'intensité du rayonnement qui est détecté dans une image spectrale telle que saisie par l'un des canaux optiques 10a, 10b,... de l'unité de saisie d'images 10. Pour cela, il peut être nécessaire de connaître les matériaux des éléments de scène qui sont contenus dans le champ optique d'entrée, pour disposer de leurs valeurs d'émissivité. Deux situations permettent de déterminer optiquement la valeur $T_{ambiante}$, en utilisant une fonction réciproque par rapport à la température, de la loi d'émission de Planck $B(\lambda, T)$, où $\lambda$ est la longueur d'onde du rayonnement détecté, T est la température du matériau qui a émis ce rayonnement, et B est la valeur détectée de brillance. La première situation est celle d'un secteur du champ optique d'entrée qui est tourné vers une zone de ciel dégagé, pour laquelle le comportement d'émission thermique de l'atmosphère est connu. En particulier, aucune quantité de gaz-cible n'y est présente, ou bien la bande spectrale utilisée est insensible au gaz-cible. La seconde situation est celle d'un secteur du champ optique d'entrée qui est occupé par une composition gazeuse connue, cette composition étant complètement opaque pour la bande spectrale utilisé. Alors des éléments de scène qui sont éventuellement présents en arrière-plan ne contribuent pas au rayonnement qui est détecté par l'unité de saisie d'images 10 pour cette bande spectrale. La composition gazeuse doit alors être connue, pour disposer de ses valeurs d'émissivité. Tel est le cas dans un secteur du champ optique d'entrée où la composition gazeuse est dépourvue de gaz-cible, et correspond donc à la composition atmosphérique. Pour de telles réalisations qui sont conformes à l'invention, où la valeur $T_{ambiante}$ est déduite d'images spectrales saisies par l'unité 10, les premiers moyens d'acquisition 21 peuvent être internes à l'unité de traitement d'images 20.

La référence 22 désigne des moyens pour obtenir et transmettre à l'unité de traitement d'images 20, des valeurs de température de brillance d'un arrière-plan qui est contenu dans le champ optique d'entrée, pour chacune des bandes spectrales. Ces moyens 22 ont été appelés seconds moyens d'acquisition dans la partie générale de la présente description. Dans des réalisations possibles de l'invention, pour lesquelles la composition de l'arrière-plan est connue initialement, les valeurs de température de brillance de cet arrière-plan peuvent être déduites, en utilisant la loi d'émission de Planck, de la valeur de température ambiante et de valeurs d'émissivité relatives à des éléments différents de l'arrière-plan. Pour cela, il est nécessaire que les surfaces de ces éléments de scène d'arrière-plan soient en équilibre thermique avec l'atmosphère. En particulier, les éléments de scène ne doivent pas être des sources de chaleur, ni recevoir de flux solaire direct. De telles réalisations des moyens d'acquisition 22 sont particulièrement adaptées lorsque le détecteur de gaz est installé fixement pour surveiller une zone dont les éléments de scène ne sont pas susceptibles d'évoluer sensiblement à l'échelle de plusieurs dizaines de secondes, en dehors d'une apparition potentielle d'une quantité de gaz-cible.

Dans d'autres réalisations possibles de l'invention, les valeurs de température de brillance de l'arrière-plan pour chaque bande spectrale peuvent être déduites d'images spectrales qui ont été saisies par l'unité 10 à un instant où aucune quantité de gaz-cible n'est présente dans le champ optique d'entrée de l'unité de saisie d'images 10. Pour de telles autres réalisations de l'invention, les moyens d'acquisition 22 peuvent être internes à l'unité de traitement d'images 20. De telles valeurs de température de brillance d'arrière-plan sont relatives séparément à chaque bande spectrale, et aussi relatives séparément à des parties différentes de l'arrière-plan tel que contenu dans le champ optique d'entrée. Par exemple, $TB_{arrière-plan\_1}(i, j)$ désigne la température de brillance de l'arrière-plan pour la bande spectrale bande_1, au point d'image de coordonnées (i, j) dans une image spectrale saisie dans la bande spectrale bande_1. Les notations $TB_{arrière-plan\_2}(i, j)$,..., $TB_{arrière-plan\_4}(i, j)$ qui sont utilisées dans la suite ont des significations identiques, respectivement pour les bandes spectrales bande_2,..., bande_4.

Toutefois, dans certaines circonstances d'utilisation d'un détecteur de gaz conforme à l'invention, il n'est pas possible de disposer d'images spectrales pour lesquelles le champ optique d'entrée soit dépourvu de gaz-cible. Tel est le cas lorsqu'il s'agit d'une fuite continue de gaz-cible qui a débuté avant que le détecteur de gaz soit apporté sur site pour confirmer l'existence de la fuite. Plusieurs méthodes sont alors possibles pour pallier une telle absence de valeurs $TB_{arrrière-plan\_1}(i, j)$,..., $TB_{arrrière-plan\_4}(i, j)$ qui soient exactes.

Selon une première de ces méthodes, une des bandes spectrales du détecteur de gaz, dite bande de référence, peut être sélectionnée pour correspondre à un domaine de transparence totale ou quasi-totale du gaz-cible et de l'atmosphère. Alors l'image spectrale qui est saisie pour cette bande de référence permet à l'unité de traitement d'images 20 de déterminer des valeurs de température de brillance qui sont relatives aux éléments de scène d'arrière-plan. Si chaque élément de scène d'arrière-plan est reconnu, par exemple en effectuant une détection de sa forme telle qu'apparente dans l'image spectrale puis en réalisant une reconnaissance de forme, son matériau peut être identifié. Alors il est possible de déterminer sa valeur de température thermodynamique d'après sa luminance dans la bande de référence, puis sa valeur de température de brillance pour toute autre bande spectrale, notamment les bandes d'analyse, à partir des variations spectrales de sa fonction d'émissivité. Chaque bande d'analyse est destinée à être utilisée ensuite pour évaluer quantitativement la quantité de gaz-cible qui est présente dans une partie du champ optique d'entrée. Par principe, chaque bande d'analyse a été choisie de sorte que le gaz-cible possède, dans cette bande d'analyse, une valeur d'absorbance de rayonnement qui est significative. Par extension de ce principe, la même méthode pour déterminer des valeurs de température de brillance d'arrière-plan peut être appliquée entre deux bandes spectrales pour lesquelles le gaz-cible possède des valeurs d'absorbance qui sont différentes : celle des deux bandes spectrales dans laquelle l'absorbance du gaz-cible est la plus faible peut être utilisée en tant que bande de référence, et l'autre bande spectrale, dans laquelle l'absorbance du gaz-cible est plus élevée, peut être utilisée en tant que bande d'analyse.

Une autre méthode possible pour pallier l'absence de valeurs $TB_{arrière-plan\_1}(i, j)$,..., $TB_{arrrière-plan\_4}(i, j)$ qui soient exactes peut être la suivante. Les valeurs de température de brillance de l'image spectrale qui a été saisie pour la bande de référence peuvent être réparties dans quelques intervalles prédéfinis et disjoints, par exemple cinq intervalles. Puis, des zones sont identifiées dans l'image spectrale de la bande de référence, pour chacun de ces intervalles, telles que dans chaque zone les valeurs de température de brillance soient contenues dans cet intervalle. Un découpage d'image est ainsi obtenu, qui est reporté dans chaque image spectrale saisie dans une bande d'analyse. Ensuite, séparément dans chaque zone du découpage, on peut déterminer des coefficients d'une régression linéaire qui relie les valeurs de température de brillance dans l'image spectrale de la bande de référence à celles dans l'image spectrale de la bande d'analyse pour la même zone de découpage. Cette régression linéaire peut alors être utilisée, pour chacun des points d'image à l'intérieur de la zone de découpage correspondante, pour convertir chaque valeur de température de brillance dans l'image spectrale de la bande de référence en une valeur de température de brillance d'arrière-plan pour la bande d'analyse, c'est-à-dire $TB_{arrrière-plan\_1}(i, j)$ si la bande d'analyse considérée est bande_1. Une telle autre méthode ne nécessite pas de mettre en oeuvre un procédé de reconnaissance de forme, ni de mémoriser les valeurs d'émissivité de matériaux possibles pour les éléments de scène d'arrière-plan. En fait, la distinction entre des éléments de scène d'arrière-plan différents qui est procurée par l'analyse de forme dans la méthode précédente, est remplacée empiriquement par la classification des valeurs de température de brillance pour la bande de référence dans des intervalles disjoints. Mais, cette autre méthode n'est fiable que si le panache de gaz-cible occupe une partie peu importante de chacune des zones de découpage. Autrement dit, le panache apparaît petit dans chaque image spectrale par rapport aux éléments de scène d'arrière-plan.

La référence 40 dans [Fig. 2] désigne une unité de stockage de données, optionnelle, dans laquelle l'unité de calcul 30 peut lire des valeurs numériques. L'unité de stockage 40 peut être constituée par tout ensemble de mémoire ou support d'inscription de données. Une utilisation d'une telle unité de stockage de données est décrite plus loin.

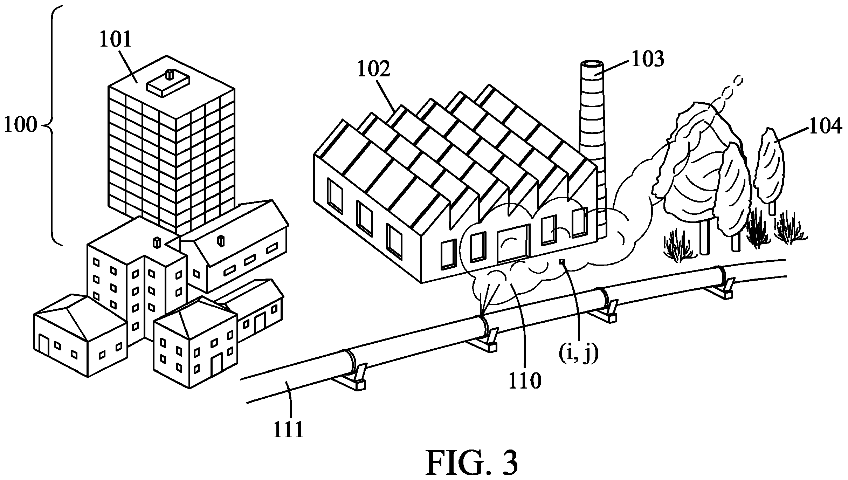

[Fig. 3] montre des circonstances possibles d'utilisation d'un détecteur de gaz conforme à l'invention. Le champ optique d'entrée de l'unité de saisie d'images 10 comprend une scène d'arrière-plan 100, et un espace libre qui est rempli de gaz atmosphérique entre cette scène d'arrière-plan 100 et l'unité de saisie d'images 10. La scène d'arrière-plan 100 peut être constituée d'éléments de scène divers, tels que des bâtiments d'habitation 101, des bâtiments industriels 102, une cheminée 103, des éléments de végétation 104, etc. L'espace libre est traversé par une canalisation de méthane 111, qui peut présenter une fuite à un endroit de cette canalisation. Cette fuite provoque un panache de méthane 110, qui est donc situé en avant de certains des éléments de la scène d'arrière-plan 100. Le but de l'invention est de révéler l'existence du panache de méthane 110 en fournissant une évaluation numérique d'une quantité de méthane qui est présente entre l'unité de saisie d'images 10 et la scène d'arrière-plan 100. (i, j) désigne un point d'image par ses coordonnées de ligne et de colonne telles qu'identifiées par le capteur d'images 2. Ce point d'image, par sa surface individuelle de détection - ou taille de pixel - et la relation d'imagerie qui est produite par chaque canal optique 10a, 10b,...de l'unité de saisie d'images 10, entre le contenu du champ optique d'entrée et le capteur d'images 2, délimite une partie du champ optique d'entrée.

Le traitement des images spectrales qui est effectué par l'unité 20 est expliqué maintenant.

Pour chaque bande spectrale dans laquelle la composition atmosphérique peut être considérée comme transparente si elle ne contient pas de gaz-cible, la luminance au point d'image (i, j) de l'image spectrale correspondante est approximativement, par exemple pour la bande bande_1 :

$$L_{bande\_1}(i, j) = L_{arrière-plan\_1}(i, j) \cdot \tau_{bande\_1} + \varepsilon_{bande\_1} \cdot B(T_{ambiante})$$

où $\tau_{bande\_1}$ est le coefficient de transmission de rayonnement du gaz-cible pour la bande spectrale concernée, i.e. bande_1, effectif au point d'image (i, j), $\varepsilon_{bande\_1}$ est le coefficient d'émissivité du gaz-cible pour cette même bande spectrale, effectif au point d'image (i, j), $L_{arrière-plan\_1}(i, j)$ est la luminance de l'élément de scène d'arrière-plan au point d'image (i, j) pour la même bande spectrale, et $B(T_{ambiante})$ est la loi d'émission de Planck. Dans cette expression de la luminance qui est saisie dans l'image spectrale par l'unité de saisie d'images 10 pour la bande spectrale bande_1, le premier terme est la contribution de l'arrière-plan 100 à travers le panache de gaz-cible 110, et le second terme est la contribution du panache de gaz-cible 110. Pour le gaz-cible : $\varepsilon_{bande\_1} = 1 - \tau_{bande\_1}$. Alors :

$$L_{bande\_1}(i, j) = L_{arrière-plan\_1}(i, j) \cdot \tau_{bande\_1} + (1 - \tau_{bande\_1}) \cdot B(T_{ambiante}),$$

ou bien :

$$L_{bande\_1}(i, j) - L_{arrière-plan\_1}(i, j) = L_{arrière-plan\_1}(i, j) \cdot (\tau_{bande\_1} - 1) + (1 - \tau_{bande\_1}) \cdot B(T_{ambiante}),$$

soit encore :

$$\tau_{bande\_1} = 1 + [L_{bande\_1}(i, j) - L_{arrière-plan\_1}(i, j)]/[L_{arrière-plan\_1}(i, j) - B(T_{ambiante})].$$

En convertissant les valeurs de luminance en valeurs de température de brillance, selon une relation affine qui est réciproque de la loi d'émission de Planck à l'intérieur d'un intervalle de valeurs de luminance et/ou de valeurs de température de brillance qui contient les valeurs utilisées, on obtient :

$$\tau_{bande\_1} = 1 + [TB_{apparente\_1}(i, j) - TB_{arrière-plan\_1}(i, j)]/[TB_{arrière-plan\_1}(i, j) - T_{ambiante}],$$

où $TB_{apparente\_1}(i, j)$ est la valeur de température de brillance apparente au point d'image (i, j) dans l'image spectrale saisie pour la bande d'analyse bande_1, $TB_{arrière-plan\_1}(i, j)$ est la valeur de température de brillance d'arrière-plan telle que fournie par les moyens d'acquisition 22, et $T_{ambiante}$ est la valeur de température ambiante telle que fournie par les moyens d'acquisition 21. La même valeur de température ambiante est utilisée pour toutes les bandes spectrales. Séparément pour chaque image spectrale qui est saisie dans une bande d'analyse k, c'est-à-dire une des bandes spectrales qui est utilisée pour détecter la présence du gaz-cible, l'unité de traitement d'images 20 convertit ainsi la valeur de luminance $L_{bande\_k}(i, j)$ en valeur de température de brillance $TB_{apparente\_k}(i, j)$, puis calcule la valeur du coefficient de transmission $\tau_{bande\_k}$ qui est attribuable au gaz-cible pour le point d'image (i, j). Il s'agit donc en fait de $\tau_{bande\_k}(i, j)$.

De façon optionnelle mais avantageuse, il est possible d'obtenir des valeurs numériques qui sont plus exactes pour les résultats de détection du gaz-cible, en corrigeant les valeurs de luminance telles que fournies par l'unité de d'images 10, d'effets d'une quantité de vapeur d'eau qui est contenue dans la composition atmosphérique entre le panache de méthane 110 et l'unité de saisie d'images 10. En effet, pour chaque bande spectrale, cette quantité de vapeur d'eau produit une contribution supplémentaire à la luminance telle que saisie par le capteur d'images 2, et atténue les contributions de l'arrière-plan 100 et du gaz-cible selon la valeur d'un coefficient spectral de transmission de rayonnement qui est relative à cette quantité de vapeur d'eau. Pour cela, des valeurs de luminance spectrale et du coefficient spectral de transmission peuvent être calculées pour la vapeur d'eau, à partir de données atmosphériques obtenues par ailleurs telles que la pression atmosphérique, le taux d'humidité relative et la température ambiante. Ces valeurs de luminance et de coefficient de transmission relatives à la vapeur d'eau sont $\tau_{H2O\ bande\_1}$ et $L_{H2O\ bande\_1}$ pour la bande spectrale bande_1,..., $\tau_{H2O\ bande\_4}$ et $L_{H2O\ bande\_4}$ pour la bande spectrale bande_4. La correction peut alors consister à remplacer chaque valeur de luminance $L_{bande\_1}(i, j)$ telle que délivrée par l'unité de saisie d'images 10, par $[L_{bande\_1}(i, j) - L_{H2O\ bande\_1}]/\tau_{H2O\ bande\_1}$ pour le calcul du coefficient de transmission du gaz-cible $\tau_{bande\_1}$, et de même pour chacune des autres bandes spectrales. La même correction peut être appliquée aux valeurs de brillance d'arrière-plan $L_{arrière-plan\_1}(i, j)$,..., $L_{arrière-plan\_4}(i, j)$ lorsque celles-ci sont utilisées pour déterminer les valeurs de température de brillance d'arrière-plan $TB_{arrière-plan\_1}(i, j)$,..., $TB_{arrière-plan\_4}(i, j)$.

Un premier critère de validation des valeurs des coefficients de transmission $\tau_{bande\_1}$,..., $\tau_{bande\_4}$ qui sont délivrées par l'unité de traitement d'images 20, peut être que chacune de ces valeurs soit comprise entre 0 et 1, ces limites 0 et 1 étant permises. Des valeurs de $\tau_{bande\_1}$,..., $\tau_{bande\_4}$ qui ne satisfont pas ce critère peuvent être écartées, et le procédé de détection peut être repris à partir de la saisie des images spectrales. Une valeur proche de 0 est attendue pour le coefficient de transmission de l'une des bandes spectrales si le gaz-cible et/ou un élément de la composition atmosphérique dont les effets sur les valeurs de luminance n'ont pas été corrigés, est très absorbant dans cette bande spectrale. A l'inverse, une valeur proche de 1 est attendue pour une bande spectrale dans laquelle le gaz-cible est peu absorbant, et s'il n'y a aucun élément de la composition atmosphérique qui soit absorbant dans cette bande spectrale et dont les effets sur les valeurs de luminance n'ont pas été corrigés.

Un deuxième critère de validation des valeurs des coefficients de transmission $\tau_{bande\_1}$,..., $\tau_{bande\_4}$ qui sont délivrées par l'unité de traitement d'images 20 peut être que ces valeurs numériques soient ordonnées conformément à un classement de valeurs de coefficients spectraux d'absorbance du gaz-cible, qui sont effectives une-à-une pour les bandes spectrales dans lesquelles le gaz-cible est plus absorbant que la composition atmosphérique.

Le fonctionnement de l'unité de calcul 30 est expliqué maintenant.

Selon une première possibilité, le détecteur de gaz peut être adapté pour déduire un résultat numérique d'évaluation de la quantité du gaz-cible séparément pour chaque bande d'analyse, et ce pour plusieurs bandes d'analyse qui sont traitées en parallèle. Les valeurs de température de brillance apparente ont été déterminées indépendamment pour toutes les bandes d'analyse, à partir d'images spectrales respectives qui ont été saisies pour ces bandes d'analyse. L'unité de traitement d'images 20 en a déduit les évaluations des coefficients de transmission de rayonnement qui sont relatifs un-à-une à ces bandes d'analyse, pour toutes les bandes d'analyse indépendamment les unes des autres. Alors, l'unité de calcul 30 peut être adaptée pour déduire des évaluations de la quantité du gaz-cible $Q_{gaz-cible}$ qui est présente dans la partie du champ optique d'entrée, par exemple au point d'image (i, j) : $Q_{gaz-cible}(i, j)$, aussi indépendamment pour chacune des bandes d'analyse, et seulement à partir de la valeur qui a été obtenue pour le coefficient de transmission de rayonnement relatif à cette bande d'analyse. Autrement dit, la quantité du gaz-cible peut être évaluée complètement et séparément à partir de chacune des bandes d'analyse, en utilisant la même méthode mais transposée à chaque bande d'analyse. Alors, une présence du gaz-cible dans la partie du champ optique d'entrée peut être affirmée, niée, ou déclarée indéterminée, en fonction d'un critère de cohérence appliqué aux valeurs qui ont été obtenues d'après toutes les bandes d'analyse pour la quantité de gaz-cible contenue dans la partie du champ optique d'entrée. De plus, une valeur plus fiable de la quantité de gaz-cible qui est présente en un point d'image peut être calculée, par exemple, comme une valeur moyenne des valeurs qui ont été obtenues séparément pour plusieurs bandes d'analyse, ou pour toutes les bandes d'analyse, pour la quantité de gaz-cible qui est présente en ce point d'image.

Toutefois, une autre possibilité de fonctionnement pour l'unité de calcul 30, qui est maintenant décrite et est préférée, peut utiliser un algorithme de sélection selon la meilleure coïncidence, ou «best match» en anglais.

De façon connue, la valeur du coefficient de transmission pour chaque bande spectrale résulte de l'intégrale sur un trajet de rayon qui parvient au point d'image spectrale (i, j), d'un facteur du type $\exp[-A_{gaz-cible} \cdot C_{gaz-cible} - \Sigma_{autre\ gaz} A_{autre\ gaz} \cdot C_{autre\ gaz}]$, où $C_{gaz-cible}$ est la concentration locale du gaz-cible en chaque point du trajet du rayon, $A_{gaz-cible}$ est l'absorbance du gaz-cible dans la bande spectrale considérée, $C_{autre\ gaz}$ est la concentration locale de chaque autre gaz présent sur le trajet du rayon, essentiellement des gaz de la composition atmosphérique dont les effets sur les valeurs de luminance saisies n'ont pas été corrigés, et $A_{autre\ gaz}$ est l'absorbance de cet autre gaz dans la bande spectrale considérée. Ainsi, l'une des bandes spectrales est d'autant plus appropriée pour évaluer la quantité du gaz-cible que la valeur d'absorbance du gaz-cible est supérieure ou très supérieure à celle de chaque autre gaz pour la bande spectrale considérée. Une telle bande spectrale appropriée a été appelée bande d'analyse dans toute la présente description.

De préférence, les bandes spectrales des canaux optiques de l'unité de saisie d'images 10 ont été sélectionnées pour qu'au moins deux d'entre elles, par exemple bande_1 et bande_2, soient des bandes d'analyse pour le gaz-cible, c'est-à-dire le méthane dans le cas présent. Par principe, les bandes d'analyse ne peuvent pas être utilisées pour déterminer la température ambiante $T_{ambiante}$, ni servir de bande de référence.

Selon une configuration préférée de l'unité de calcul 30, celle-ci peut être en communication de données avec l'unité de stockage 40, et une table a été enregistrée initialement dans cette dernière, qui contient des valeurs pré-calculées pour les coefficients de transmission de chaque bande d'analyse : $\tau_{bande\_1}$ et $\tau_{bande\_2}$ dans l'exemple présent. Ces valeurs ont été calculées pour des profils de concentration variables du gaz-cible le long d'un trajet de rayon, correspondant à des dilutions locales variables du gaz-cible dans la composition atmosphérique. Les valeurs spectrales d'absorbance $A_{gaz-cible}$ et $A_{autre\ gaz}$ qui sont utilisées pour ces calculs ont été déterminées par des méthodes d'évaluation spectroscopiques, connues par ailleurs de l'Homme du métier. Alors, pour chaque point d'image (i, j) qui est commun à au moins deux images spectrales de bandes d'analyse, l'unité de calcul 30 sélectionne celui des profils de concentration du gaz-cible le long du trajet de rayon, pour lequel les valeurs pré-calculées des coefficients de transmission pour ces bandes d'analyse coïncident le mieux, numériquement, avec les valeurs fournies par l'unité de traitement d'images 20 pour ces mêmes coefficients de transmission. Un critère de validation supplémentaire de la détection du gaz-cible peut être qu'un même profil de concentration du gaz-cible le long du trajet de rayon permette de rendre compte avec une exactitude suffisante, des valeurs des coefficients de transmission qui ont été déduites des images spectrales simultanément pour plusieurs bandes d'analyse.

Une fois que le profil de concentration du gaz-cible le long du trajet de rayon qui arrive au point d'image (i, j) a été déterminé, l'unité de calcul 30 en déduit une valeur de la quantité du gaz-cible $Q_{gaz-cible}$ qui est présente sur ce trajet de rayon. Eventuellement, pour chaque profil de concentration du gaz-cible, le résultat de la quantité du gaz-cible qui est présente sur le trajet de rayon peut aussi avoir été pré-calculé, et enregistré dans l'unité de stockage 40 avec les valeurs des coefficients de transmission de rayonnement qui correspondent à ce profil de concentration pour les bandes d'analyse. Eventuellement, cette quantité peut être intégrée sur tous les points d'image pour lesquels des profils de concentration non nuls ont été déterminés, afin d'évaluer le panache 110 dans son entièreté, à l'intérieur du champ optique d'entrée.

Il est entendu que l'invention peut être reproduite en modifiant ou adaptant des aspects secondaires des modes de réalisation qui ont été décrits en détail ci-dessus, tout en conservant certains au moins des avantages cités. Notamment, les adaptations suivantes sont possibles :

- les bandes spectrales peuvent être sélectionnées pour détecter un gaz autre que le méthane, par exemple pour détecter des quantités de sulfure d'hydrogène ($H_2S$), notamment telles que pouvant être produites par des algues en décomposition sur une plage, ou encore pour détecter un gaz d'industrie chimique échappé accidentellement ;
- les équations et formules qui ont été citées, notamment pour calculer le coefficient de transmission de rayonnement pour chaque bande d'analyse, ou pour la correction des effets de la vapeur d'eau contenue dans la composition atmosphérique sur les valeurs de luminance saisies, ou pour déduire la quantité de gaz-cible à partir de chaque coefficient de transmission de rayonnement relatif à une bande d'analyse, peuvent être remplacées par des équations ou formules équivalentes ou sensiblement équivalentes ;
- les valeurs de luminance qui sont utilisées pour déduire celles des coefficients de transmission du gaz-cible pour les bandes d'analyse, peuvent être déterminées chacune pour une partie du champ optique d'entrée de l'unité de saisie d'images, qui correspond à plusieurs points d'image d'adjacents dans les images spectrales, au lieu d'être relatives chacune à un seul point d'image ;
- les valeurs de luminance qui sont utilisées par l'unité de traitement d'images pour obtenir les valeurs des coefficients de transmission peuvent être corrigées des effets de composants de l'atmosphère autres que la valeur d'eau, par exemple des effets du dioxyde de carbone, en fonction des bandes spectrales et de la composition d'atmosphère sur le site d'utilisation du détecteur de gaz ; et
- toutes les valeurs numériques qui ont été citées ne l'ont été qu'à titre d'illustration, et peuvent être changées en fonction de l'application considérée.

**Revendications**

1.  Détecteur de gaz, pour révéler au moins un gaz appelé gaz-cible et qui est susceptible d'être présent dans un champ optique d'entrée, ledit détecteur de gaz comprenant :

    - une unité de saisie d'images (10), qui comprend au moins deux canaux optiques (10a, 10b,...) disposés en parallèle pour saisir séparément et simultanément des images respectives d'un même contenu du champ optique d'entrée, appelées images spectrales, dans des bandes spectrales différentes auxquelles lesdits canaux sont dédiés un-à-une ;

- des premiers moyens d'acquisition (21), adaptés pour fournir une valeur de température ambiante, destinée à être attribuée à une quantité du gaz-cible qui est présente dans le champ optique d'entrée entre une scène d'arrière-plan et l'unité de saisie d'images (10) ;

- des seconds moyens d'acquisition (22), adaptés pour fournir, pour au moins une des bandes spectrales appelée bande d'analyse, des valeurs de température de brillance d'arrière-plan qui sont destinées à être attribuées à des éléments de la scène d'arrière-plan ;

- une unité de traitement d'images (20), agencée pour recevoir les images spectrales saisies par chaque canal (10a, 10b,...) de l'unité de saisie d'images (10), et adaptée pour déduire, séparément pour chaque bande d'analyse, une évaluation d'un coefficient de transmission de rayonnement qui est relatif à ladite bande d'analyse, et qui est attribué au moins partiellement à une quantité du gaz-cible présente dans une partie du champ optique d'entrée entre les éléments de scène d'arrière-plan et l'unité de saisie d'images, à partir d'une équation qui combine :

    . la valeur de température ambiante,
    . la valeur de température de brillance d'arrière-plan attribuée aux éléments de scène d'arrière-plan dans la partie du champ optique d'entrée, pour ladite bande d'analyse, et
    . au moins une valeur de température de brillance, appelée valeur de température de brillance apparente, qui correspond à l'image spectrale saisie pour la bande d'analyse, dans ladite partie du champ optique d'entrée ; et

- une unité de calcul (30), adaptée pour déduire une évaluation de la quantité de gaz-cible qui est présente dans la partie du champ optique d'entrée, à partir de la valeur du coefficient de transmission de rayonnement qui est relatif à la bande d'analyse,

le détecteur de gaz étant **caractérisé en ce que** les premiers moyens d'acquisition (21) sont adaptés pour fournir la valeur de température ambiante selon l'une des façons suivantes :

- à partir d'au moins une partie d'une image spectrale qui a été saisie par un des canaux (10a, 10b,...) de l'unité de saisie d'images (10), ladite partie d'image spectrale correspondant à un secteur du champ optique d'entrée déclaré ou considéré comme étant dépourvu d'éléments de scène d'arrière-plan ; et

- à partir d'au moins une partie d'une image spectrale qui a été saisie par un des canaux (10a, 10b,...) de l'unité de saisie d'images (10), dont la bande spectrale associée est contenue dans un domaine spectral d'opacité complète d'un gaz qui est présent dans un secteur du champ optique d'entrée correspondant à la partie d'image spectrale,

et **en ce que** le détecteur de gaz est adapté pour saisir l'image spectrale dont une partie au moins est utilisée pour fournir la valeur de température ambiante, en même temps ou
lors d'une même séquence d'acquisition d'image, que l'image spectrale de chaque bande d'analyse lorsque l'unité de traitement d'images (20) combine ensuite ladite valeur de température ambiante avec au moins une valeur de température de brillance de ladite image spectrale de bande d'analyse.

2. Détecteur de gaz selon la revendication 1, dans lequel l'unité de traitement d'images (20) est adaptée pour évaluer le coefficient de transmission du rayonnement qui est relatif à la bande d'analyse, et qui est attribué au moins partiellement au gaz-cible, selon l'équation :

$$\tau_{bande\_1} = 1 + (TB_{apparente\_1} - TB_{arrière\text{-}plan\_1})/(TB_{arrière\text{-}plan\_1} - T_{ambiante})$$

où :

$\tau_{bande\_1}$ est la valeur du coefficient de transmission du rayonnement qui est relatif à la bande d'analyse, notée bande_1, et qui est attribué au moins partiellement au gaz-cible présent dans la partie du champ optique d'entrée,
$T_{ambiante}$ est la valeur de température ambiante qui est fournie par les premiers moyens d'acquisition (21),
$TB_{arrière\text{-}plan\_1}$ est la valeur de température de brillance d'arrière-plan qui est fournie par les seconds moyens d'acquisition (22), et qui est attribuée aux éléments de scène d'arrière-plan contenus dans la partie du champ optique d'entrée, pour la bande d'analyse bande_1, et
$TB_{apparente\_1}$ est la valeur de température de brillance apparente qui correspond à l'image spectrale saisie pour la bande d'analyse bande_1, dans la partie du champ optique d'entrée.

**3.** Détecteur de gaz selon la revendication 1 ou 2, dans lequel l'unité de traitement d'images (20) est adaptée en outre pour appliquer à chaque image spectrale, une correction de valeurs de luminance telles que saisies par l'unité de saisie d'images (10), afin de prendre en compte au moins un composant atmosphérique qui est présent dans le champ optique d'entrée, et pour utiliser les valeurs de luminance corrigées pour déduire l'évaluation du coefficient de transmission du rayonnement relatif à chaque bande d'analyse.

**4.** Détecteur de gaz selon l'une quelconque des revendications précédentes, dans lequel l'unité de calcul (30) est agencée pour déterminer la quantité du gaz-cible présente dans la partie du champ optique d'entrée en comparant les valeurs des coefficients de transmission de rayonnement qui ont été déduites par l'unité de traitement d'images (20) séparément pour plusieurs bandes d'analyse, à des valeurs desdits coefficients de transmission de rayonnement prédéterminées pour les mêmes bandes d'analyse, et enregistrées dans une unité de stockage de données (40) accessible par l'unité de calcul, lesdites valeurs prédéterminées et enregistrées étant relatives à des profils variables d'une concentration du gaz-cible sur un trajet de rayonnement qui relie un élément de scène d'arrière-plan à l'unité de saisie d'images (10).

**5.** Détecteur de gaz selon l'une quelconque des revendications précédentes, dans lequel les seconds moyens d'acquisition (22) sont adaptés pour fournir les valeurs de température de brillance d'arrière-plan selon l'une des façons suivantes :

- à partir d'au moins une image spectrale qui contient un des éléments de scène d'arrière-plan, qui a été saisie par le canal de l'unité de saisie d'images (10) dédié à la bande d'analyse, et qui est déclarée ou considérée comme ayant été saisie alors que le champ optique d'entrée ne contenait pas de gaz-cible ;
- à partir d'au moins une image spectrale qui contient un des éléments de scène d'arrière-plan, qui a été saisie par un des canaux (10a, 10b,...) de l'unité de saisie d'images (10) pour lequel la bande spectrale correspondante, dite bande de référence, est contenue dans un domaine spectral de transparence du gaz-cible, ou dans laquelle bande de transparence le gaz-cible possède une transparence plus grande que dans chaque bande d'analyse, l'unité de traitement d'images (20) étant adaptée pour identifier un matériau de l'élément de scène d'arrière-plan à partir de ladite image spectrale saisie pour la bande de référence, et pour déduire la valeur de température de brillance d'arrière-plan pour chaque bande d'analyse et pour ledit élément de scène d'arrière-plan, à partir de la valeur de température ambiante et d'une valeur d'émissivité spectrale du matériau identifié pour ledit élément de scène d'arrière-plan ; et
- à partir d'au moins une image spectrale qui contient un des éléments de scène d'arrière-plan, qui a été saisie par un des canaux (10a, 10b,...) de l'unité de saisie d'images (10) pour lequel la bande spectrale correspondante, dite bande de référence, est contenue dans un domaine spectral de transparence du gaz-cible, ou dans laquelle bande de transparence le gaz-cible possède une transparence plus grande que dans chaque bande d'analyse, l'unité de traitement d'images (20) étant adaptée pour déduire les valeurs de température de brillance d'arrière-plan pour chaque bande d'analyse en utilisant une régression linéaire à partir de valeurs de température de brillance d'arrière-plan déduites de l'image spectrale saisie pour la bande de référence.

**6.** Détecteur de gaz selon la revendication 5, dans lequel les seconds moyens d'acquisition (22) sont adaptés en outre pour fournir les valeurs de température de brillance d'arrière-plan à partir d'au moins une image spectrale saisie par un des canaux (10a, 10b,...) de l'unité de saisie d'images (10) pour lequel la bande spectrale correspondante, dite bande de référence, est contenue dans un domaine spectral de transparence du gaz-cible, ou dans laquelle bande de transparence le gaz-cible possède une transparence plus grande que dans chaque bande d'analyse, le détecteur de gaz étant adapté pour saisir l'image spectrale qui correspond à la bande de référence en même temps ou lors d'une même séquence d'acquisition d'image, que l'image spectrale de chaque bande d'analyse.

**7.** Détecteur de gaz selon l'une quelconque des revendications précédentes, dans lequel l'unité de saisie d'images (10) comprend un capteur d'images matriciel (2) qui est commun à tous les canaux (10a, 10b,...), et qui est sensible simultanément dans toutes les bandes spectrales desdits canaux, avec une partie (Sa, Sb) d'une surface photosensible (S) dudit capteur d'images qui est dédiée à chaque canal séparément de chaque autre canal, chaque canal (10a, 10b,...) comprenant, à l'intérieur de l'unité de saisie d'images (10) :

- une partie d'optique (1a, 1b) qui est agencée pour former une image d'un contenu du champ optique d'entrée sur la partie de surface photosensible (Sa, Sb) dédiée audit canal, le champ optique d'entrée étant commun à tous les canaux ; et
- des moyens de filtrage spectral (3a, 3b), qui sont adaptés pour déterminer la bande spectrale dudit canal.

**8.** Détecteur de gaz selon l'une quelconque des revendications précédentes, dans lequel chaque canal (10a, 10b,...) de l'unité de saisie d'images (10) comprend des moyens de filtrage spectral (3a, 3b) tels que la bande spectrale dudit canal possède une largeur qui est comprise entre 10 nm et 500 nm, en termes de longueur d'onde du rayonnement.

**9.** Détecteur de gaz selon la revendication 8, dans lequel les moyens de filtrage spectral (3a, 3b) sont adaptés pour que les bandes spectrales des canaux (10a, 10b,...) soient contenues dans un premier domaine spectral correspondant à des longueurs d'onde de rayonnement comprises entre 7 $\mu$m et 10 $\mu$m, ou contenues dans un second domaine spectral correspondant à des longueurs d'onde de rayonnement comprises entre 3 $\mu$m et 5 $\mu$m.

**10.** Détecteur de gaz selon la revendication 9, adapté pour le méthane en tant que gaz-cible, et dans lequel :

- la bande spectrale d'un premier des canaux (10a, 10b,...) s'étend autour de 7,7 $\mu$m, avec une largeur de bande spectrale qui est inférieure à 0,35 $\mu$m ; et
- la bande spectrale d'un deuxième des canaux (10a, 10b,...) s'étend autour de 8,05 $\mu$m, avec une largeur de bande spectrale qui est inférieure à 0,35 $\mu$m ;

et optionnellement :

- la bande spectrale d'un troisième des canaux (10a, 10b,...) s'étend autour de 7,35 $\mu$m, avec une largeur de bande spectrale qui est inférieure à 0,35 $\mu$m ; et
- la bande spectrale d'un quatrième des canaux (10a, 10b,...) s'étend autour de 8,35 $\mu$m ou 9,05 $\mu$m, avec une largeur de bande spectrale qui est inférieure à 0,35 $\mu$m.

**11.** Détecteur de gaz selon la revendication 9, adapté pour le méthane en tant que gaz-cible, et dans lequel :

- la bande spectrale d'un premier des canaux (10a, 10b,...) s'étend autour de 3,375 $\mu$m, avec une largeur de bande spectrale qui est inférieure à 0,30 $\mu$m ; et
- la bande spectrale d'un deuxième des canaux (10a, 10b,...) s'étend autour de 3,225 $\mu$m, avec une largeur de bande spectrale qui est inférieure à 0,30 $\mu$m ;

et optionnellement :

- la bande spectrale d'un troisième des canaux (10a, 10b,...) s'étend autour de 3,05 $\mu$m, avec une largeur de bande spectrale qui est inférieure à 0,30 $\mu$m ; et
- la bande spectrale d'un quatrième des canaux (10a, 10b,...) s'étend autour de 4,237 $\mu$m ou 3,505 $\mu$m, avec une largeur de bande spectrale qui est inférieure à 0,30 $\mu$m.

**12.** Détecteur de gaz selon l'une quelconque des revendications précédentes, adapté pour combiner les images spectrales d'un même contenu du champ optique d'entrée qui sont saisies séparément par deux des canaux optiques (10a, 10b,...) de l'unité de saisie d'images (10), dits canaux optiques élémentaires, afin d'obtenir une image spectrale qui correspond à une bande spectrale d'un canal optique composite, la bande spectrale du canal optique composite résultant de positions relatives et de largeurs spectrales des bandes spectrales respectives des deux canaux optiques élémentaires.

**13.** Détecteur de gaz selon l'une quelconque des revendications précédentes, adapté pour révéler plusieurs gaz-cibles différents qui sont susceptibles d'être présents simultanément dans le même champ optique d'entrée, et dans lequel la bande spectrale de l'un au moins des canaux (10a, 10b,...) de l'unité de saisie d'images (10) est contenue simultanément dans des domaines spectraux d'absorption respectifs d'au moins deux des gaz-cibles, de sorte qu'une même image spectrale qui est saisie par ledit canal soit utilisée par l'unité de traitement d'images (20) et l'unité de calcul (30) pour déduire des évaluations de quantités respectives desdits au moins deux gaz-cibles qui sont présentes dans le champ optique d'entrée.

**Patentansprüche**

**1.** Gasdetektor zum Erkennen wenigstens eines Gases, das als Zielgas bezeichnet wird und in einem optischen Eingangsfeld vorhanden sein kann, wobei der Gasdetektor umfasst:

- eine Bilderfassungseinheit (10), die wenigstens zwei parallel angeordnete optische Kanäle (10a, 10b,...) umfasst, um getrennt und gleichzeitig jeweilige Bilder desselben Inhalts des optischen Eingangsfeldes, sogenannte Spektralbilder, in verschiedenen Spektralbändern zu erfassen, denen die Kanäle jeweils zugeordnet sind;

- erste Erfassungsmittel (21), die dazu ausgebildet sind, einen Umgebungstemperaturwert vorzusehen, der dazu bestimmt ist, einer Menge des Zielgases zugeordnet zu werden, das sich im optischen Eingangsfeld zwischen einer Hintergrundszene und der Bilderfassungseinheit (10) befindet;

- zweite Erfassungsmittel (22), die dazu ausgebildet sind, für wenigstens eines der Spektralbänder, das sogenannte Analyseband, Werte der Hintergrundhelligkeitstemperatur vorzusehen, die dazu bestimmt sind, Elementen der Hintergrundszene zugeordnet zu werden;

- eine Bildverarbeitungseinheit (20), die dazu angeordnet ist, die von jedem Kanal (10a, 10b,... ) der Bilderfassungseinheit (10) erfassten Spektralbilder zu empfangen und dazu ausgebildet ist, separat für jedes Analyseband eine Evaluation eines Strahlungstransmissionskoeffizienten abzuleiten, der sich auf das Analyseband bezieht und der wenigstens teilweise einer Menge des Zielgases zugeordnet ist, das sich in einem Teil des optischen Eingangsfeldes zwischen den Hintergrundszenenelementen und der Bilderfassungseinheit befindet, basierend auf einer Gleichung, die kombiniert:

• den Umgebungstemperaturwert,

• den Hintergrundhelligkeitstemperaturwert, der den Hintergrundszeneelementen in dem Teil des optischen Eingangsfelds für das Analyseband zugeordnet ist, und

• wenigstens einen Helligkeitstemperaturwert, den sogenannten sichtbaren Helligkeitstemperaturwert, der dem für das Analyseband erfassten Spektralbild in dem Teil des optischen Eingangsfeldes entspricht; und

- eine Recheneinheit (30), die dazu ausgebildet ist, eine Evaluation der Menge des Zielgases, die in dem Teil des optischen Eintrittsfeldes vorhanden ist, basierend auf dem Wert des Strahlungstransmissionskoeffizienten, der sich auf das Analyseband bezieht, abzuleiten,

wobei der Gasdetektor **dadurch gekennzeichnet ist, dass** die ersten Erfassungsmittel (21) dazu ausgebildet sind, den Umgebungstemperaturwert auf eine der folgenden Weisen vorzusehen:

- basierend auf wenigstens einem Teil eines Spektralbildes, das von einem der Kanäle (10a, 10b,...) der Bilderfassungseinheit (10) erfasst wurde, wobei der Teil des Spektralbildes einem Sektor des optischen Eingangsfeldes entspricht, der als frei von Hintergrundszenenelementen deklariert oder betrachtet wird; und

- basierend auf wenigstens einem Teil eines Spektralbildes, das von einem der Kanäle (10a, 10b,... ) der Bilderfassungseinheit (10) erfasst wurde, deren zugeordnetes Spektralband in einem Spektralbereich der vollständigen Lichtundurchlässigkeit eines Gases enthalten ist, das in einem Abschnitt des optischen Eingangsfeldes vorhanden ist, der dem Teil des Spektralbildes entspricht,

und dass der Gasdetektor dazu ausgebildet ist, das Spektralbild zu erfassen, von dem wenigstens ein Teil dazu verwendet wird, den Wert der Umgebungstemperatur vorzusehen, zur gleichen Zeit oder in der gleichen Bilderfassungssequenz, wie das Spektralbild jedes Analysebandes, wenn die Bildverarbeitungseinheit (20) dann den Umgebungstemperaturwert mit wenigstens einem Helligkeitstemperaturwert des Spektralbildes des Analysebandes kombiniert.

**2.** Gasdetektor nach Anspruch 1, wobei die Bildverarbeitungseinheit (20) dazu ausgebildet ist, den Transmissionskoeffizienten der Strahlung, der sich auf das Analyseband bezieht und der wenigstens teilweise dem Zielgas zugeschrieben wird, nach der Gleichung

$$\tau_{bande\_1} = 1 + (TB_{apparente\_1} - TB_{arrière\text{-}plan\_1})/(TB_{arrière\text{-}plan\_1} - T_{ambiante})$$

auszuwerten,
wobei:

$\tau_{bande\_1}$ der Wert des Strahlungstransmissionskoeffizienten ist, der sich auf das Analyseband bezieht, mit bande_1 bezeichnet, und der wenigstens teilweise dem Zielgas zugeschrieben wird, das in dem Teil des optischen Eintrittsfeldes vorliegt,

$T_{ambiante}$ der Umgebungstemperaturwert ist, der von den ersten Erfassungsmitteln (21) vorgesehen wird,

$TB_{arrière\text{-}plan\_1}$ der Hintergrundhelligkeitstemperaturwert ist, der von den zweiten Erfassungsmitteln (22) vorgesehen wird und der den in dem Teil des optischen Eintrittsfeldes enthaltenen Hintergrundszenenelementen für das Analyseband bande_1 zugeordnet wird, und

$TB_{apparent\_1}$ der sichtbare Helligkeitstemperaturwert ist, der dem für das Analyseband bande_1 in dem Teil des optischen Eintrittsfeldes erfassten Spektralbild entspricht.

3. Gasdetektor nach Anspruch 1 oder 2, wobei die Bildverarbeitungseinheit (20) ferner dazu ausgebildet ist, auf jedes Spektralbild eine Korrektur der Leuchtdichtewerte, wie sie von der Bilderfassungseinheit (10) erfasst werden, anzuwenden, um wenigstens eine atmosphärische Komponente zu berücksichtigen, die im optischen Eintrittsfeld vorhanden ist, und um die korrigierten Leuchtdichtewerte zu verwenden, um eine Evaluation des Strahlungstransmissionskoeffizienten in Bezug auf jedes Analyseband abzuleiten.

4. Gasdetektor nach einem der vorhergehenden Ansprüche, wobei die Recheneinheit (30) dazu angeordnet ist, die Menge des in dem Teil des optischen Eintrittsfeldes vorhandenen Zielgases durch Vergleich der Werte der von der Bildverarbeitungseinheit (20) separat für mehrere Analysebänder abgeleiteten Strahlungstransmissionskoeffizienten mit Werten der Strahlungstransmissionskoeffizienten zu bestimmen, die für die gleichen Analysebänder vorbestimmt und in einer Datenspeichereinheit (40) gespeichert sind, auf die die Recheneinheit zugreifen kann, wobei sich die vorbestimmten und gespeicherten Werte auf variable Profile einer Konzentration des Zielgases auf einem Strahlungsweg beziehen, der ein Hintergrundszenenelement mit der Bilderfassungseinheit (10) verbindet.

5. Gasdetektor nach einem der vorhergehenden Ansprüche, wobei die zweiten Erfassungsmittel (22) dazu ausgebildet sind, die Hintergrundhelligkeitstemperaturwerte auf eine der folgenden Weisen vorzusehen:

   - basierend auf wenigstens einem Spektralbild, das eines der Hintergrundszenenelemente umfasst, das von dem Kanal der Bilderfassungseinheit (10) erfasst wurde, der dem Analyseband zugeordnet ist, und von dem erklärt oder angenommen wird, dass es erfasst wurde, während das optische Eintrittsfeld kein Zielgas enthielt;
   - basierend auf wenigstens einem Spektralbild, das eines der Hintergrundszenenelemente umfasst, das von einem der Kanäle (10a, 10b,...) der Bilderfassungseinheit (10) erfasst wurde, für die das entsprechende Spektralband, das sogenannte Referenzband, in einem Transparenzspektralbereich des Zielgases enthalten ist, oder in welchem Transparenzband das Zielgas eine größere Transparenz aufweist als in jedem Analyseband, wobei die Bildverarbeitungseinheit (20) dazu ausgebildet ist, ein Material des Hintergrundszenenelements basierend auf dem für das Referenzband erfassten Spektralbild zu identifizieren, und um den Hintergrundhelligkeitstemperaturwert für jedes Analyseband und für das Hintergrundszenelement basierend auf dem Umgebungstemperaturwert und einem spektralen Emissionsgradwert des identifizierten Materials für das Hintergrundszenelement abzuleiten; und
   - basierend auf wenigstens einem Spektralbild, welches eines der Hintergrundszenenelemente umfasst, welches von einem der Kanäle (10a, 10b,...) der Bilderfassungseinheit (10) erfasst wurde, für die das entsprechende Spektralband, das sogenannte Referenzband, in einem Transparenzspektralbereich des Zielgases enthalten ist, oder in welchem Transparenzband das Zielgas eine größere Transparenz besitzt als in jedem Analyseband, wobei die Bildverarbeitungseinheit (20) dazu ausgebildet ist, die Hintergrundhelligkeitstemperaturwerte für jedes Analyseband unter Verwendung einer linearen Regression basierend auf den Hintergrundhelligkeitstemperaturwerten abzuleiten, die basierend auf dem für das Referenzband erfassten Spektralbild abgeleitet wurden.

6. Gasdetektor nach Anspruch 5, wobei die zweiten Erfassungsmittel (22) zusätzlich dazu ausgebildet sind, die Hintergrundhelligkeitstemperaturwerte basierend auf wenigstens einem Spektralbild vorzusehen, das von einem der Kanäle (10a, 10b,...) der Bilderfassungseinheit (10) erfasst wird, für den das entsprechende Spektralband, das sogenannte Referenzband, in einem Transparenzspektralbereich des Zielgases enthalten ist, oder in welchem Transparenzband das Zielgas eine größere Transparenz besitzt als in jedem Analyseband,
   wobei der Gasdetektor dazu ausgebildet ist, das dem Referenzband entsprechende Spektralbild zur gleichen Zeit oder in der gleichen Bildaufnahmesequenz wie das Spektralbild jedes Analysebandes zu erfassen.

7. Gasdetektor nach einem der vorhergehenden Ansprüche, wobei die Bilderfassungseinheit (10) einen Matrixbildsensor (2) umfasst, der allen Kanälen (10a, 10b,...) gemeinsam ist und der gleichzeitig in allen Spektralbändern der Kanäle sensitiv ist, mit einem Teil (Sa, Sb) einer lichtempfindlichen Fläche (S) des Bildsensors, der jedem Kanal getrennt von jedem anderen Kanal zugeordnet ist,
   wobei jeder Kanal (10a, 10b,...) innerhalb der Bilderfassungseinheit (10) umfasst:

   - einen optischen Teil (1a, 1b), der dazu angeordnet ist, ein Bild eines Inhalts des optischen Eingangsfeldes auf dem Teil der lichtempfindlichen Oberfläche (Sa, Sb) zu bilden, der dem Kanal zugeordnet ist, wobei das optische Eingangsfeld allen Kanälen gemeinsam ist; und
   - Spektralfiltermittel (3a, 3b), die dazu ausgebildet sind, das Spektralband des Kanals zu bestimmen.

8. Gasdetektor nach einem der vorhergehenden Ansprüche, wobei jeder Kanal (10a, 10b,...) der Bilderfassungseinheit (10) Spektralfiltermittel (3a, 3b) umfasst, so dass das Spektralband des Kanals eine Breite aufweist, die in Bezug auf die Wellenlänge der Strahlung zwischen 10 nm und 500 nm liegt.

9. Gasdetektor nach Anspruch 8, wobei die Spektralfiltermittel (3a, 3b) derart ausgebildet sind, dass die Spektralbänder der Kanäle (10a, 10b,...) in einem ersten Spektralbereich enthalten sind, der Strahlungswellenlängen zwischen 7 $\mu$m und 10 $\mu$m entspricht, oder in einem zweiten Spektralbereich enthalten sind, der Strahlungswellenlängen zwischen 3 $\mu$m und 5 $\mu$m entspricht.

10. Gasdetektor nach Anspruch 9, ausgebildet für Methan als Zielgas, und wobei:

- sich das Spektralband eines ersten der Kanäle (10a, 10b,...) um 7,7 $\mu$m erstreckt, mit einer Spektralbandbreite, die kleiner als 0,35 $\mu$m ist; und
- sich das Spektralband eines zweiten der Kanäle (10a, 10b,...) um 8,05 $\mu$m erstreckt, mit einer Spektralbandbreite, die kleiner als 0,35 $\mu$m ist;

und optional:

- sich das Spektralband eines dritten der Kanäle (10a, 10b,...) um 7,35 $\mu$m erstreckt, mit einer Spektralbandbreite, die kleiner als 0,35 $\mu$m ist; und
- sich das Spektralband eines vierten der Kanäle (10a, 10b,...) um 8,35 $\mu$m oder 9,05 $\mu$m erstreckt, mit einer Spektralbandbreite, die kleiner als 0,35 $\mu$m ist.

11. Gasdetektor nach Anspruch 9, ausgebildet für Methan als Zielgas, und wobei:

- sich das Spektralband eines ersten der Kanäle (10a, 10b,...) um 3,375 $\mu$m erstreckt, mit einer Spektralbandbreite, die kleiner als 0,30 $\mu$m ist; und
- sich das Spektralband eines zweiten der Kanäle (10a, 10b,...) um 3,225 $\mu$m erstreckt, mit einer Spektralbandbreite, die kleiner als 0,30 $\mu$m ist;

und optional:

- sich das Spektralband eines dritten der Kanäle (10a, 10b,...) um 3,05 $\mu$m erstreckt, mit einer Spektralbandbreite, die kleiner als 0,30 $\mu$m ist; und
- sich das Spektralband eines vierten der Kanäle (10a, 10b,...) um 4,237 $\mu$m oder 3,505 $\mu$m erstreckt, mit einer Spektralbandbreite, die kleiner als 0,30 $\mu$m ist.

12. Gasdetektor nach einem der vorhergehenden Ansprüche, dazu ausgebildet Spektralbilder desselben Inhalts des optischen Eintrittsfeldes zu kombinieren, die getrennt von zwei der optischen Kanäle (10a, 10b,...) der Bilderfassungseinheit (10), den sogenannten elementaren optischen Kanälen, erfasst werden, um ein Spektralbild zu erhalten, das einem Spektralband eines zusammengesetzten optischen Kanals entspricht, wobei das Spektralband des zusammengesetzten optischen Kanals aus den relativen Positionen und spektralen Breiten der jeweiligen Spektralbänder der beiden elementaren optischen Kanäle resultiert.

13. Gasdetektor nach einem der vorhergehenden Ansprüche, dazu ausgebildet, mehrere verschiedene Zielgase aufzudecken, die gleichzeitig in demselben optischen Eingangsfeld vorhanden sein können, und wobei das Spektralband von wenigstens einem der Kanäle (10a, 10b,...) der Bilderfassungseinheit (10) gleichzeitig in den jeweiligen Absorptionsspektralbereichen von wenigstens zwei der Zielgase enthalten ist, so dass dasselbe Spektralbild, das von diesem Kanal erfasst wird, von der Bildverarbeitungseinheit (20) und der Recheneinheit (30) verwendet wird, um Schätzungen der jeweiligen Mengen der wenigstens zwei Zielgase abzuleiten, die in dem optischen Eintrittsfeld vorhanden sind.

**Claims**

1. A gas detector for revealing at least one gas which is called target gas and which could be present in an optical field-of-view, where said gas detector comprises:

- an image capturing unit (10), which comprises at least two optical channels (10a, 10b,...) arranged in parallel for separately and simultaneously capturing respective images of one and same content of the optical field-of-view, called spectral images, in different spectral bands to which said channels are dedicated one-to-one;

- first acquisition means (21) adapted for providing an ambient temperature value, intended to be assigned to a quantity of the target gas which is present in the optical field-of-view between a background scene and the image capturing unit (10);

- second acquisition means (22), adapted for providing, for at least one of the spectral bands called analysis band, background brightness temperature values which are intended to be assigned to elements of the background scene;

- an image processing unit (20), arranged for the receiving spectral images captured by each channel (10a, 10b,...) of the image capturing unit (10), and adapted for deducing, separately for each analysis band, and evaluation of a radiation transmission coefficient which relates to said analysis band, and which is assigned at least partially to a quantity of the target gas present in a part of the optical field-of-view between the background scene elements and the image capturing unit, based on an equation which combines:

  - the ambient temperature value;
  - the background brightness temperature value assigned to the background scene elements in the part of the optical field-of-view, for said analysis band; and
  - at least one brightness temperature value, called apparent brightness temperature value, which corresponds to the spectral image captured for the analysis band, in said part of the optical field-of-view; and

- a calculation unit (30), adapted for deducing an evaluation of the quantity of the target gas which is present in the optical field-of-view, based on the value of the radiation transmission coefficient which relates to the analysis band,

wherein the gas detector is **characterized in that** the first acquisition means (21) are adapted for providing the ambient temperature value according to one of the following ways:

- based on at least one part of a spectral image which was captured by one of the input channels (10a, 10b,...) of the image capturing unit (10), said spectral image part corresponding to a sector of the optical field-of-view declared or considered as being free of background scene elements; and

- based on at least one part of a spectral image which was captured by one of the channels (10a, 10b,...) of the image capturing unit (10), whose associated spectral band is contained in a spectral domain of complete opacity for a gas which is present in a sector of the optical field-of-view corresponding to the spectral image portion;

and **in that**, the gas detector is adapted for capturing the spectral image at least a part of which is used in order to provide the ambient temperature value, at a same time or during a same image capturing sequence as the spectral image of each analysis band, when the image processing unit (20) then combines said ambient temperature value with at least one brightness temperature value of said spectral image of analysis band.

2. The gas detector according to claim 1, wherein the image processing unit (20) is adapted for evaluating the radiation transmission coefficient which relates to the analysis band, and which is assigned at least partially to the target gas, according to the equation:

$$\tau_{band\_1} = 1 + (TB_{apparent\_1} - TB_{background\_1})/(TB_{background\_1} - T_{ambient})$$

where:

$\tau_{band\_1}$ is the value of the transmission coefficient for the radiation which relates to the analysis band, indicated by band_1, and which is assigned at least partially to the target gas present in the part of the optical field-of-view;

$T_{ambient}$ is the ambient temperature value which is provided by the first acquisition means (21);

$TB_{background\_1}$ is the background brightness temperature value, which is provided by the second acquisition means (22), and which is assigned to the background scene elements contained in the part of the optical field-of-view, for the analysis band band_1; and

$TB_{apparent\_1}$ is the apparent brightness temperature value, which corresponds to the spectral image captured for the analysis band band_1, in the part of the optical field-of-view.

3. The gas detector according to claim 1 or 2, wherein the image processing unit (20) is further adapted for applying to each spectral image a correction of luminance values as captured by the image capturing unit (10), in order to take into account at least one atmospheric compound which is present in the optical field-of-view, and for using the corrected luminance values for deducing the evaluation of the radiation transmission coefficient relating to each analysis band.

4. The gas detector according to any one of the preceding claims, wherein the calculation unit (30) is arranged for determining the quantity of target gas which is present in the part of the optical field-of-view by comparing the values of the radiation transmission coefficients which were deduced by the image processing unit (20) separately for multiple analysis bands, with values of said radiation transmission coefficients predetermined for the same analysis bands, and recorded in a data storage unit (40) accessible to the calculation unit, wherein said predetermined and recorded values relate to variable profiles of a concentration of the target gas on a radiation path which connects a background scene element to the image capturing unit (10).

5. The gas detector according to any one of the preceding claims, wherein the second acquisition means (22) are adapted for providing the background brightness temperature values according to one of the following ways:

- from at least one spectral image which contains one of the background scene elements, which was captured by the channel of the image capturing unit (10) dedicated to the analysis band, and which is declared or considered as having been captured when the optical field-of-view did not contain target gas;
- from at least one spectral image which contains one of the background scene elements, which was captured by one of the channels (10a, 10b,...) of the image capturing unit (10) for which the corresponding spectral band, called reference band, is contained in a spectral domain of transparency of the target gas, or in which transparency band the target gas has a transparency greater than in each analysis band, wherein the image processing unit (20) is adapted for identifying a material of the background scene element from said spectral image captured for the reference band, and for deducing the background brightness temperature value for each analysis band and for said background scene element, based on the ambient temperature value and a spectral emissivity value of the identified material, for said background scene element; and
- from at least one spectral image which contains one of the background scene elements, which was captured by one of the channels (10a, 10b,...) of the image capturing unit (10) for which the corresponding spectral band, called reference band, is contained in a spectral domain of transparency of the target gas, or in which transparency band the target gas has a greater transparency than in each analysis band, wherein the image processing unit (20) is adapted for producing the background brightness temperature values for each analysis band by using a linear regression based on background brightness temperature values deduced from the spectral image captured for the reference band.

6. The gas detector according to claim 5, wherein the second acquisition means (22) are further adapted for providing the background brightness temperature values based on at least one spectral image captured by one of the channels (10a, 10b,...) of the image capturing unit (10) for which the corresponding spectral band, called reference band, is contained in a spectral domain of transparency of the target gas, or in which transparency band the target gas has a greater transparency than in each analysis band,
wherein the gas detector is adapted for capturing the spectral image which corresponds to the reference band at a same time or during a same image capturing sequence as the spectral image for each analysis band.

7. The gas detector according to any one of the preceding claims wherein the image capturing unit (10) comprises a matrix image sensor (2) which is common to all the channels (10a, 10b,...), and which is simultaneously sensitive in all the spectral bands of said channels, with a part (Sa, Sb) of a photosensitive surface (S) of said image sensor that is dedicated to each channel separately from each other channel,
each channel (10a, 10b,...) comprising, inside the image capturing unit (10):

- an optical part (1a, 1b) which is arranged in order to form an image of a content of the optical field-of-view on the part (Sa, Sb) on the photosensitive surface part dedicated to said channel, wherein the optical field-of-view is common to all the channels; and
- spectral filtering means (3a, 3b), which are adapted for determining the spectral band of said channel.

8. The gas detector according to any one of the preceding claims, wherein each channel (10a, 10b,...) of the image capturing unit (10) comprises spectral filtering means (3a, 3b) such that the spectral band of said channel has a width which is comprised between 10 nm and 500 nm, in terms of wavelength of the radiation.

9. The gas detector according to claim 8, wherein the spectral filtering means (3a, 3b) are adapted so that the spectral bands of the channels (10a, 10b,...) are contained in a first spectral domain which corresponds to radiation wavelengths comprised between 7 $\mu$m and 10 $\mu$m, or contained in a second spectral domain which corresponds to radiation wavelengths comprised between 3 $\mu$m and 5 $\mu$m.

10. The gas detector according to claim 9, adapted for methane as the target gas, and wherein:

   - the spectral band of a first of the channels (10a, 10b,...) extends around 7.7 $\mu$m, with a spectral bandwidth which is less than 0.35 $\mu$m; and
   - the spectral band of a second of the channels (10a, 10b,...) extends around 8.05 $\mu$m, with a spectral bandwidth which is less than 0.35 $\mu$m;

   and optionally:

   - the spectral band of a third of the channels (10a, 10b,...) extends around 7.35 $\mu$m, with a spectral bandwidth which is less than 0.35 $\mu$m; and
   - the spectral band of a fourth of the channels (10a, 10b,...) extends around 8.35 $\mu$m or 9.05 $\mu$m, with a spectral bandwidth which is less than 0.35 $\mu$m.

11. The gas detector according to claim 9, adapted for methane as the target gas, and wherein:

   - the spectral band of a first of the channels (10a, 10b,...) extends around 3.375 $\mu$m, with a spectral bandwidth which is less than 0.30 $\mu$m; and
   - the spectral band of a second of the channels (10a, 10b,...) extends around 3.225 $\mu$m, with a spectral bandwidth which is less than 0.30 $\mu$m;

   and optionally:

   - the spectral band of a third of the channels (10a, 10b,...) extends around 3.05 $\mu$m, with a spectral bandwidth which is less than 0.30 $\mu$m; and
   - the spectral band of a fourth of the channels (10a, 10b,...) extends around 4.237 $\mu$m or 3.505 $\mu$m, with a spectral bandwidth which is less than 0.30 $\mu$m.

12. The gas detector according to any one of the preceding claims, adapted for combining the spectral images of a same content of the optical field-of-view which are captured separately by two of the optical channels (10a, 10b,...) of the image capturing unit (10), called base optical channels, in order to get a spectral image which corresponds to a spectral band of a composite optical channel, wherein the spectral band of the composite optical channel results from relative positions and spectral bandwidths of the respective spectral bands of the two base optical channels.

13. The gas detector according to any one of the preceding claims, adapted for revealing several different target gases which could be simultaneously present in the same optical field-of-view; and wherein the spectral band of at least one of the channels (10a, 10b,...) of the image capturing unit (10) is simultaneously contained in respective spectral absorption domains of at least two of the target gases, so that one same spectral image which is captured by said channel is used by the image processing unit (20) and the calculation unit (30) for deducing evaluations of respective quantities of said at least two target gases which are present in the optical field-of-view.

FIG. 1

EP 3 953 683 B1

$image_{bande\_1}$

$\vdots$

$image_{bande\_4}$

10a

10b

$\underline{10}$

$\underline{20}$

$\underline{21}$ $\underline{22}$

$\tau_{bande\_1}$

$\vdots$

$\tau_{bande\_4}$

$\underline{30}$

$Q_{gaz\_cible}$

$T_{ambiante}$

$TB_{arrière\text{-}plan\_1}$

$\vdots$

$TB_{arrière\text{-}plan\_4}$

$\boxed{\underline{21}}$ $\boxed{\underline{22}}$

$\underline{40}$

# FIG. 2

FIG. 3

**EP 3 953 683 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2016097713 A

- US 2018011009 A